# EUROPEAN PATENT APPLICATION

(11) **EP 1 408 049 A2**
(43) Date of publication of application: **14.04.2004**
(21) Application number: 03256457.7
(22) Date of filing: 13.10.2003
(51) Int. Cl.: C07K 14/00

(54) **A novel inositol 1,4,5-trisphosphate (IP3) receptor-binding protein and an IP3 indicator**

(30) Priority: 11.10.2002 JP 2002299429
(71) Applicant: Riken, Wako-shi, Saitama 351-0198 (JP)
(72) Inventor: Mikoshiba, Katsuhiko, Wako-shi, Saitama 351-0198 (JP); Ando, Hideaki, Wako-shi, Saitama 351-0198 (JP); Mizutani, Akihiro, Wako-shi, Saitama 351-0198 (JP); Matsu-Ura, Toru, Tokyo 152-0002 (JP)
(74) Representative: Maschio, Antonio, Dr.

(57) **Abstract**

The present invention provides a novel IP₃ receptor-binding protein that is used as an indicator effective for detecting and quantifying IP₃, and a method for detecting and/or quantifying IP₃ using the same. Determined is an IP₃ receptor-binding protein, particularly a novel IP₃ receptor-binding protein, IRBIT, which interacts with an IP₃ receptor, and whose interaction is regulated by IP₃. IRBIT has the characteristic that it can be dissociated from the IP₃ receptor by IP₃ at a physiological concentration. Because of this characteristic, IRBIT can be useful as an indicator for detecting IP₃ by the FRET method or the like.

SEQUENCE LISTING

<110> RIKEN

<120> A novel inositol 1,4,5-trisphosphate (IP3) receptor binding protein and an IP3
indicator

<130> PH-1852

<150> JP 2002-299429
<151> 2002-10-11

<160> 7

<170> PatentIn Ver. 2.1

<210> 1
<211> 530
<212> PRT
<213> Mus musculus

<400> 1
Met Ser Met Pro Asp Ala Met Pro Leu Pro Gly Val Gly Glu Glu Leu
  1               5                  10                  15

Lys Gln Ala Lys Glu Ile Glu Asp Ala Glu Lys Tyr Ser Phe Met Ala
             20                  25                  30

Thr Val Thr Lys Ala Pro Lys Lys Gln Ile Gln Phe Ala Asp Asp Met
         35                  40                  45

Gln Glu Phe Thr Lys Phe Pro Thr Lys Thr Gly Arg Arg Ser Leu Ser
    50                  55                  60
Arg Ser Ile Ser Gln Ser Ser Thr Asp Ser Tyr Ser Ser Ala Ala Ser
65                  70                  75                  80
Tyr Thr Asp Ser Ser Asp Asp Glu Val Ser Pro Arg Glu Lys Gln Gln
                85                  90                  95
Thr Asn Ser Lys Gly Ser Ser Asn Phe Cys Val Lys Asn Ile Lys Gln
            100                 105                 110
Ala Glu Phe Gly Arg Arg Glu Ile Glu Ile Ala Glu Gln Asp Met Ser
        115                 120                 125
Ala Leu Ile Ser Leu Arg Lys Arg Ala Gln Gly Glu Lys Pro Leu Ala
    130                 135                 140
Gly Ala Lys Ile Val Gly Cys Thr His Ile Thr Ala Gln Thr Ala Val
145                 150                 155                 160
Leu Ile Glu Thr Leu Cys Ala Leu Gly Ala Gln Cys Arg Trp Ser Ala
                165                 170                 175
Cys Asn Ile Tyr Ser Thr Gln Asn Glu Val Ala Ala Ala Leu Ala Glu
            180                 185                 190
Ala Gly Val Ala Val Phe Ala Trp Lys Gly Glu Ser Glu Asp Asp Phe
        195                 200                 205

Trp Trp Cys Ile Asp Arg Cys Val Asn Met Asp Gly Trp Gln Ala Asn
    210                 215                 220

Met Ile Leu Asp Asp Gly Gly Asp Leu Thr His Trp Val Tyr Lys Lys
225                 230                 235                 240

Tyr Pro Asn Val Phe Lys Lys Ile Arg Gly Ile Val Glu Glu Ser Val
                245                 250                 255

Thr Gly Val His Arg Leu Tyr Gln Leu Ser Lys Ala Gly Lys Leu Cys
            260                 265                 270

Val Pro Ala Met Asn Val Asn Asp Ser Val Thr Lys Gln Lys Phe Asp
        275                 280                 285

Asn Leu Tyr Cys Cys Arg Glu Ser Ile Leu Asp Gly Leu Lys Arg Thr
    290                 295                 300

Thr Asp Val Met Phe Gly Gly Lys Gln Val Val Val Cys Gly Tyr Gly
305                 310                 315                 320

Glu Val Gly Lys Gly Cys Cys Ala Ala Leu Lys Ala Leu Gly Ala Ile
                325                 330                 335

Val Tyr Ile Thr Glu Ile Asp Pro Ile Cys Ala Leu Gln Ala Cys Met
            340                 345                 350

Asp Gly Phe Arg Val Val Lys Leu Asn Glu Val Ile Arg Gln Val Asp
        355                 360                 365

Val Val Ile Thr Cys Thr Gly Asn Lys Asn Val Val Thr Arg Glu His
    370                 375                 380

Leu Asp Arg Met Lys Asn Ser Cys Ile Val Cys Asn Met Gly His Ser
385                 390                 395                 400

Asn Thr Glu Ile Asp Val Thr Ser Leu Arg Thr Pro Glu Leu Thr Trp
                405                 410                 415

Glu Arg Val Arg Ser Gln Val Asp His Val Ile Trp Pro Asp Gly Lys
            420                 425                 430

Arg Val Val Leu Leu Ala Glu Gly Arg Leu Leu Asn Leu Ser Cys Ser
        435                 440                 445

Thr Val Pro Thr Phe Val Leu Ser Ile Thr Ala Thr Thr Gln Ala Leu
    450                 455                 460

Ala Leu Ile Glu Leu Tyr Asn Ala Pro Glu Gly Arg Tyr Lys Gln Asp
465                 470                 475                 480

Val Tyr Leu Leu Pro Lys Lys Met Asp Glu Tyr Val Ala Ser Leu His
                485                 490                 495

Leu Pro Ser Phe Asp Ala His Leu Thr Glu Leu Thr Asp Asp Gln Ala
            500                 505                 510

Lys Tyr Leu Gly Leu Asn Lys Asn Gly Pro Phe Lys Pro Asn Tyr Tyr
        515                 520                 525

Arg Tyr
    530

<210> 2
<211> 1593
<212> DNA
<213> Mus musculus

<400> 2
atgtcgatgc ctgacgcgat gccgctgccc ggtgtcgggg aggagctgaa acaggccaag 60
gagatcgagg acgccgagaa gtactccttc atggccacgg tcaccaaggc tcccaagaag 120
caaatccagt ttgctgatga catgcaagag ttcaccaaat tccctactaa gactggccgg 180
agatctttgt ctcgttccat ctcacaatcc tccacagaca gctacagttc agctgcatcc 240
tatacagata gctctgatga tgaggtttcc cctcgagaga agcagcaaac caactcgaag 300
ggcagcagca atttctgtgt gaagaacatc aagcaggcag agtttggacg ccgggagatt 360
gagattgcag agcaagacat gtctgctctg atttcactca ggaaacgtgc tcagggagag 420
aagcctttgg ctggtgctaa aatagtgggc tgtacgcaca tcacggccca gacagcggta 480
ttaattgaga ccctttgtgc cctgggagct cagtgccgct ggtctgcctg caacatctat 540
tcaactcaga atgaagtagc tgcagcactg gctgaggctg gagtcgcggt gtttgcttgg 600
aagggcgagt cagaagatga tttctggtgg tgcattgacc gctgtgtcaa catggatggg 660
tggcaggcta acatgatcct ggatgatggg ggagacttaa cccactgggt ttataagaag 720
tatccaaacg tgtttaagaa gatccgaggc attgtggaag agagcgtgac tggtgttcac 780
aggctgtatc agctctccaa agctgggaag ctctgtgttc cagccatgaa tgtcaatgat 840
tctgttacca aacagaagtt tgataacctg tactgctgcc gagaatccat tttggatggc 900
ctgaagagga ccacggatgt gatgtttggt gggaaacagg tggtggtgtg tggctatggt 960
gaggtaggaa agggctgctg tgctgctctc aaggcccttg gagcaattgt ctacataaca 1020
gaaattgacc ccatctgtgc tctgcaggcc tgcatggatg ggttcagggt ggtgaagctg 1080
aatgaagtca tccggcaggt ggacgttgta attacttgca caggaaataa gaatgtagtg 1140

actcgggagc acttggaccg aatgaaaaat agttgtattg tgtgcaatat gggccattcc 1200
aacacggaga tcgacgtgac cagcctccgc actccagaac taacatggga gcgtgtacgt 1260
tctcaggtgg accatgtcat ctggcctgat ggcaaacggg tcgtccttct agcagagggc 1320
cgtttactta atctgagctg ctccacagtc cctacctttg ttctttccat cacggctaca 1380
acacaggctt tggcactgat agagctttac aacgccccgg agggacgcta caaacaggat 1440
gtgtacttgc ttcctaagaa gatggatgaa tatgttgcca gcttgcactt accatcattt 1500
gatgcccacc tgacagaact gacagatgac caagcaaagt atctgggact caacaaaaat 1560
gggccattca aacctaatta ttacagatac taa 1593

<210> 3
<211> 9
<212> PRT
<213> Mus musculus

<400> 3
Tyr Ser Phe Met Ala Thr Val Thr Lys
1               5

<210> 4
<211> 13
<212> PRT
<213> Mus musculus

<400> 4
Gln Ile Gln Phe Ala Asp Asp Met Gln Glu Phe Thr Lys
1               5                   10

<210> 5

<211> 22

<212> DNA

<213> Artificial

<220>

<223> Description of Artificial Sequence: a primer for mouse IRBIT

<400> 5

atgtcgatgc ctgacgcgat gc                                                22

<210> 6

<211> 22

<212> DNA

<213> Artificial

<220>

<223> Description of Artificial Sequence: a primer for mouse IRBIT

<400> 6

gcgtggttca tgtggactgg tc                                                22

<210> 7

<211> 2749

<212> PRT

<213> Mus musculus

<400> 7

Met Ser Asp Lys Met Ser Ser Phe Leu His Ile Gly Asp Ile Cys Ser
1               5                   10                  15

Leu Tyr Ala Glu Gly Ser Thr Asn Gly Phe Ile Ser Thr Leu Gly Leu
            20                  25                  30

Val Asp Asp Arg Cys Val Val Gln Pro Glu Ala Gly Asp Leu Asn Asn
        35                  40                  45

Pro Pro Lys Lys Phe Arg Asp Cys Leu Phe Lys Leu Cys Pro Met Asn
    50                  55                  60

Arg Tyr Ser Ala Gln Lys Gln Phe Trp Lys Ala Ala Lys Pro Gly Ala
65                  70                  75                  80

Asn Ser Thr Thr Asp Ala Val Leu Leu Asn Lys Leu His His Ala Ala
                85                  90                  95

Asp Leu Glu Lys Lys Gln Asn Glu Thr Glu Asn Arg Lys Leu Leu Gly
            100                 105                 110

Thr Val Ile Gln Tyr Gly Asn Val Ile Gln Leu Leu His Leu Lys Ser
        115                 120                 125

Asn Lys Tyr Leu Thr Val Asn Lys Arg Leu Pro Ala Leu Leu Glu Lys
    130                 135                 140

Asn Ala Met Arg Val Thr Leu Asp Glu Ala Gly Asn Glu Gly Ser Trp
145                 150                 155                 160

Phe Tyr Ile Gln Pro Phe Tyr Lys Leu Arg Ser Ile Gly Asp Ser Val
                165                 170                 175

Val Ile Gly Asp Lys Val Val Leu Asn Pro Val Asn Ala Gly Gln Pro
            180                 185                 190

Leu His Ala Ser Ser His Gln Leu Val Asp Asn Pro Gly Cys Asn Glu
        195                 200                 205

Val Asn Ser Val Asn Cys Asn Thr Ser Trp Lys Ile Val Leu Phe Met
    210                 215                 220

Lys Trp Ser Asp Asn Lys Asp Asp Ile Leu Lys Gly Gly Asp Val Val
225                 230                 235                 240

Arg Leu Phe His Ala Glu Gln Glu Lys Phe Leu Thr Cys Asp Glu His
                245                 250                 255

Arg Lys Lys Gln His Val Phe Leu Arg Thr Thr Gly Arg Gln Ser Ala
            260                 265                 270

Thr Ser Ala Thr Ser Ser Lys Ala Leu Trp Glu Val Glu Val Val Gln
        275                 280                 285

His Asp Pro Cys Arg Gly Gly Ala Gly Tyr Trp Asn Ser Leu Phe Arg
    290                 295                 300

Phe Lys His Leu Ala Thr Gly His Tyr Leu Ala Ala Glu Val Asp Pro
305                 310                 315                 320

Asp Phe Glu Glu Glu Cys Leu Glu Phe Gln Pro Ser Val Asp Pro Asp
                325                 330                 335
Gln Asp Ala Ser Arg Ser Arg Leu Arg Asn Ala Gln Glu Lys Met Val
            340                 345                 350
Tyr Ser Leu Val Ser Val Pro Glu Gly Asn Asp Ile Ser Ser Ile Phe
        355                 360                 365
Glu Leu Asp Pro Thr Thr Leu Arg Gly Gly Asp Ser Leu Val Pro Arg
    370                 375                 380
Asn Ser Tyr Val Arg Leu Arg His Leu Cys Thr Asn Thr Trp Val His
385                 390                 395                 400
Ser Thr Asn Ile Pro Ile Asp Lys Glu Glu Glu Lys Pro Val Met Leu
                405                 410                 415
Lys Ile Gly Thr Ser Pro Leu Lys Glu Asp Lys Glu Ala Phe Ala Ile
            420                 425                 430
Val Pro Val Ser Pro Ala Glu Val Arg Asp Leu Asp Phe Ala Asn Asp
        435                 440                 445
Ala Ser Lys Val Leu Gly Ser Ile Ala Gly Lys Leu Glu Lys Gly Thr
    450                 455                 460
Ile Thr Gln Asn Glu Arg Arg Ser Val Thr Lys Leu Leu Glu Asp Leu
465                 470                 475                 480

Val Tyr Phe Val Thr Gly Gly Thr Asn Ser Gly Gln Asp Val Leu Glu
                485                 490                 495

Val Val Phe Ser Lys Pro Asn Arg Glu Arg Gln Lys Leu Met Arg Glu
            500                 505                 510

Gln Asn Ile Leu Lys Gln Ile Phe Lys Leu Leu Gln Ala Pro Phe Thr
        515                 520                 525

Asp Cys Gly Asp Gly Pro Met Leu Arg Leu Glu Glu Leu Gly Asp Gln
    530                 535                 540

Arg His Ala Pro Phe Arg His Ile Cys Arg Leu Cys Tyr Arg Val Leu
545                 550                 555                 560

Arg His Ser Gln Gln Asp Tyr Arg Lys Asn Gln Glu Tyr Ile Ala Lys
                565                 570                 575

Gln Phe Gly Phe Met Gln Lys Gln Ile Gly Tyr Asp Val Leu Ala Glu
            580                 585                 590

Asp Thr Ile Thr Ala Leu Leu His Asn Asn Arg Lys Leu Leu Glu Lys
        595                 600                 605

His Ile Thr Ala Ala Glu Ile Asp Thr Phe Val Ser Leu Val Arg Lys
    610                 615                 620

Asn Arg Glu Pro Arg Phe Leu Asp Tyr Leu Ser Asp Leu Cys Val Ser
625                 630                 635                 640

Met Asn Lys Ser Ile Pro Val Thr Gln Glu Leu Ile Cys Lys Ala Val
                645                 650                 655

Leu Asn Pro Thr Asn Ala Asp Ile Leu Ile Glu Thr Lys Leu Val Leu
            660                 665                 670

Ser Arg Phe Glu Phe Glu Gly Val Ser Thr Gly Glu Asn Ala Leu Glu
        675                 680                 685

Ala Gly Glu Asp Glu Glu Glu Val Trp Leu Phe Trp Arg Asp Ser Asn
    690                 695                 700

Lys Glu Ile Arg Ser Lys Ser Val Arg Glu Leu Ala Gln Asp Ala Lys
705                 710                 715                 720

Glu Gly Gln Lys Glu Asp Arg Asp Ile Leu Ser Tyr Tyr Arg Tyr Gln
                725                 730                 735

Leu Asn Leu Phe Ala Arg Met Cys Leu Asp Arg Gln Tyr Leu Ala Ile
            740                 745                 750

Asn Glu Ile Ser Gly Gln Leu Asp Val Asp Leu Ile Leu Arg Cys Met
        755                 760                 765

Ser Asp Glu Asn Leu Pro Tyr Asp Leu Arg Ala Ser Phe Cys Arg Leu
    770                 775                 780

Met Leu His Met His Val Asp Arg Asp Pro Gln Glu Gln Val Thr Pro
785                 790                 795                 800

Val Lys Tyr Ala Arg Leu Trp Ser Glu Ile Pro Ser Glu Ile Ala Ile
                805                 810                 815
Asp Asp Tyr Asp Ser Ser Gly Thr Ser Lys Asp Glu Ile Lys Glu Arg
            820                 825                 830
Phe Ala Gln Thr Met Glu Phe Val Glu Glu Tyr Leu Arg Asp Val Val
        835                 840                 845
Cys Gln Arg Phe Pro Phe Ser Asp Lys Glu Lys Asn Lys Leu Thr Phe
    850                 855                 860
Glu Val Val Asn Leu Ala Arg Asn Leu Ile Tyr Phe Gly Phe Tyr Asn
865                 870                 875                 880
Phe Ser Asp Leu Leu Arg Leu Thr Lys Ile Leu Leu Ala Ile Leu Asp
                885                 890                 895
Cys Val His Val Thr Thr Ile Phe Pro Ile Ser Lys Met Thr Lys Gly
            900                 905                 910
Glu Glu Asn Lys Gly Ser Asn Val Met Arg Ser Ile His Gly Val Gly
        915                 920                 925
Glu Leu Met Thr Gln Val Val Leu Arg Gly Gly Gly Phe Leu Pro Met
    930                 935                 940
Thr Pro Met Ala Ala Ala Pro Glu Gly Asn Val Lys Gln Ala Glu Pro
945                 950                 955                 960

Glu Lys Glu Asp Ile Met Val Met Asp Thr Lys Leu Lys Ile Ile Glu
                965                 970                 975

Ile Leu Gln Phe Ile Leu Asn Val Arg Leu Asp Tyr Arg Ile Ser Cys
            980                 985                 990

Leu Leu Cys Ile Phe Lys Arg Glu Phe Asp Glu Ser Asn Ser Gln Ser
        995                 1000                1005

Ser Glu Thr Ser Ser Gly Asn Ser Ser Gln Glu Gly Pro Ser Asn Val
    1010                1015                1020

Pro Gly Ala Leu Asp Phe Glu His Ile Glu Glu Gln Ala Glu Gly Ile
1025                1030                1035                1040

Phe Gly Gly Ser Glu Glu Asn Thr Pro Leu Asp Leu Asp Asp His Gly
                1045                1050                1055

Gly Arg Thr Phe Leu Arg Val Leu Leu His Leu Thr Met His Asp Tyr
            1060                1065                1070

Pro Pro Leu Val Ser Gly Ala Leu Gln Leu Leu Phe Arg His Phe Ser
        1075                1080                1085

Gln Arg Gln Glu Val Leu Gln Ala Phe Lys Gln Val Gln Leu Leu Val
    1090                1095                1100

Thr Ser Gln Asp Val Asp Asn Tyr Lys Gln Ile Lys Gln Asp Leu Asp
1105                1110                1115                1120

Gln Leu Arg Ser Ile Val Glu Lys Ser Glu Leu Trp Val Tyr Lys Gly
                1125                1130                1135

Gln Gly Pro Asp Glu Pro Met Asp Gly Ala Ser Gly Glu Asn Glu His
            1140                1145                1150

Lys Lys Thr Glu Glu Gly Thr Ser Lys Pro Leu Lys His Glu Ser Thr
        1155                1160                1165

Ser Ser Tyr Asn Tyr Arg Val Val Lys Glu Ile Leu Ile Arg Leu Ser
    1170                1175                1180

Lys Leu Cys Val Gln Glu Ser Ala Ser Val Arg Lys Ser Arg Lys Gln
1185                1190                1195                1200

Gln Gln Arg Leu Leu Arg Asn Met Gly Ala His Ala Val Val Leu Glu
                1205                1210                1215

Leu Leu Gln Ile Pro Tyr Glu Lys Ala Glu Asp Thr Lys Met Gln Glu
            1220                1225                1230

Ile Met Arg Leu Ala His Glu Phe Leu Gln Asn Phe Cys Ala Gly Asn
        1235                1240                1245

Gln Gln Asn Gln Ala Leu Leu His Lys His Ile Asn Leu Phe Leu Lys
    1250                1255                1260

Pro Gly Ile Leu Glu Ala Val Thr Met Gln His Ile Phe Met Asn Asn
1265                1270                1275                1280

Phe Gln Leu Cys Ser Glu Ile Asn Glu Arg Val Val Gln His Phe Val
                1285                1290                1295

His Cys Ile Glu Thr His Gly Arg Asn Val Gln Tyr Ile Lys Phe Leu
            1300                1305                1310

Gln Thr Ile Val Lys Ala Glu Gly Lys Phe Ile Lys Lys Cys Gln Asp
        1315                1320                1325

Met Val Met Ala Glu Leu Val Asn Ser Gly Glu Asp Val Leu Val Phe
    1330                1335                1340

Tyr Asn Asp Arg Ala Ser Phe Gln Thr Leu Ile Gln Met Met Arg Ser
1345                1350                1355                1360

Glu Arg Asp Arg Met Asp Glu Asn Ser Pro Leu Met Tyr His Ile His
                1365                1370                1375

Leu Val Glu Leu Leu Ala Val Cys Thr Glu Gly Lys Asn Val Tyr Thr
            1380                1385                1390

Glu Ile Lys Cys Asn Ser Leu Leu Pro Leu Asp Asp Ile Val Arg Val
        1395                1400                1405

Val Thr His Glu Asp Cys Ile Pro Glu Val Lys Ile Ala Tyr Ile Asn
    1410                1415                1420

Phe Leu Asn His Cys Tyr Val Asp Thr Glu Val Glu Met Lys Glu Ile
1425                1430                1435                1440

Tyr Thr Ser Asn His Met Trp Lys Leu Phe Glu Asn Phe Leu Val Asp
                1445                1450                1455

Ile Cys Arg Ala Cys Asn Asn Thr Ser Asp Arg Lys His Ala Asp Ser
            1460                1465                1470

Ile Leu Glu Lys Tyr Val Thr Glu Ile Val Met Ser Ile Val Thr Thr
        1475                1480                1485

Phe Phe Ser Ser Pro Phe Ser Asp Gln Ser Thr Thr Leu Gln Thr Arg
    1490                1495                1500

Gln Pro Val Phe Val Gln Leu Leu Gln Gly Val Phe Arg Val Tyr His
1505                1510                1515                1520

Cys Asn Trp Leu Met Pro Ser Gln Lys Ala Ser Val Glu Ser Cys Ile
                1525                1530                1535

Arg Val Leu Ser Asp Val Ala Lys Ser Arg Ala Ile Ala Ile Pro Val
            1540                1545                1550

Asp Leu Asp Ser Gln Val Asn Asn Leu Phe Leu Lys Ser His Asn Ile
        1555                1560                1565

Val Gln Lys Thr Ala Leu Asn Trp Arg Leu Ser Ala Arg Asn Ala Ala
    1570                1575                1580

Arg Arg Asp Ser Val Leu Ala Ala Ser Arg Asp Tyr Arg Asn Ile Ile
1585                1590                1595                1600

Glu Arg Leu Gln Asp Ile Val Ser Ala Leu Glu Asp Arg Leu Arg Pro
                1605                1610                1615
Leu Val Gln Ala Glu Leu Ser Val Leu Val Asp Val Leu His Arg Pro
            1620                1625                1630
Glu Leu Leu Phe Pro Glu Asn Thr Asp Ala Arg Arg Lys Cys Glu Ser
        1635                1640                1645
Gly Gly Phe Ile Cys Lys Leu Ile Lys His Thr Lys Gln Leu Leu Glu
    1650                1655                1660
Glu Asn Glu Glu Lys Leu Cys Ile Lys Val Leu Gln Thr Leu Arg Glu
1665                1670                1675                1680
Met Met Thr Lys Asp Arg Gly Tyr Gly Glu Lys Gln Ile Ser Ile Asp
                1685                1690                1695
Glu Ser Glu Asn Ala Glu Leu Pro Gln Ala Pro Glu Ala Glu Asn Ser
            1700                1705                1710
Thr Glu Gln Glu Leu Glu Pro Ser Pro Pro Leu Arg Gln Leu Glu Asp
        1715                1720                1725
His Lys Arg Gly Glu Ala Leu Arg Gln Ile Leu Val Asn Arg Tyr Tyr
    1730                1735                1740
Gly Asn Ile Arg Pro Ser Gly Arg Arg Glu Ser Leu Thr Ser Phe Gly
1745                1750                1755                1760

Asn Gly Pro Leu Ser Pro Gly Gly Pro Ser Lys Pro Gly Gly Gly Gly
                1765                1770                1775

Gly Gly Pro Gly Ser Ser Ser Thr Ser Arg Gly Glu Met Ser Leu Ala
            1780                1785                1790

Glu Val Gln Cys His Leu Asp Lys Glu Gly Ala Ser Asn Leu Val Ile
        1795                1800                1805

Asp Leu Ile Met Asn Ala Ser Ser Asp Arg Val Phe His Glu Ser Ile
    1810                1815                1820

Leu Leu Ala Ile Ala Leu Leu Glu Gly Gly Asn Thr Thr Ile Gln His
1825                1830                1835                1840

Ser Phe Phe Cys Arg Leu Thr Glu Asp Lys Lys Ser Glu Lys Phe Phe
                1845                1850                1855

Lys Val Phe Tyr Asp Arg Met Lys Val Ala Gln Gln Glu Ile Lys Ala
            1860                1865                1870

Thr Val Thr Val Asn Thr Ser Asp Leu Gly Asn Lys Lys Lys Asp Asp
        1875                1880                1885

Glu Val Asp Arg Asp Ala Pro Ser Arg Lys Lys Ala Lys Glu Pro Thr
    1890                1895                1900

Thr Gln Ile Thr Glu Glu Val Arg Asp Gln Leu Leu Glu Ala Ser Ala
1905                1910                1915                1920

Ala Thr Arg Lys Ala Phe Thr Thr Phe Arg Arg Glu Ala Asp Pro Asp
                1925                1930                1935
Asp His Tyr Gln Ser Gly Glu Gly Thr Gln Ala Thr Thr Asp Lys Ala
            1940                1945                1950
Lys Asp Asp Leu Glu Met Ser Ala Val Ile Thr Ile Met Gln Pro Ile
        1955                1960                1965
Leu Arg Phe Leu Gln Leu Leu Cys Glu Asn His Asn Arg Asp Leu Gln
    1970                1975                1980
Asn Phe Leu Arg Cys Gln Asn Asn Lys Thr Asn Tyr Asn Leu Val Cys
1985                1990                1995                2000
Glu Thr Leu Gln Phe Leu Asp Cys Ile Cys Gly Ser Thr Thr Gly Gly
                2005                2010                2015
Leu Gly Leu Leu Gly Leu Tyr Ile Asn Glu Lys Asn Val Ala Leu Ile
            2020                2025                2030
Asn Gln Thr Leu Glu Ser Leu Thr Glu Tyr Cys Gln Gly Pro Cys His
        2035                2040                2045
Glu Asn Gln Asn Cys Ile Ala Thr His Glu Ser Asn Gly Ile Asp Ile
    2050                2055                2060
Ile Thr Ala Leu Ile Leu Asn Asp Ile Asn Pro Leu Gly Lys Lys Arg
2065                2070                2075                2080

Met Asp Leu Val Leu Glu Leu Lys Asn Asn Ala Ser Lys Leu Leu Leu
                2085                2090                2095

Ala Ile Met Glu Ser Arg His Asp Ser Glu Asn Ala Glu Arg Ile Leu
            2100                2105                2110

Tyr Asn Met Arg Pro Lys Glu Leu Val Glu Val Ile Lys Lys Ala Tyr
        2115                2120                2125

Met Gln Gly Glu Val Glu Phe Glu Asp Gly Glu Asn Gly Glu Asp Gly
    2130                2135                2140

Ala Ala Ser Pro Arg Asn Val Gly His Asn Ile Tyr Ile Leu Ala His
2145                2150                2155                2160

Gln Leu Ala Arg His Asn Lys Glu Leu Gln Thr Met Leu Lys Pro Gly
                2165                2170                2175

Gly Gln Val Asp Gly Asp Glu Ala Leu Glu Phe Tyr Ala Lys His Thr
            2180                2185                2190

Ala Gln Ile Glu Ile Val Arg Leu Asp Arg Thr Met Glu Gln Ile Val
        2195                2200                2205

Phe Pro Val Pro Ser Ile Cys Glu Phe Leu Thr Lys Glu Ser Lys Leu
    2210                2215                2220

Arg Ile Tyr Tyr Thr Thr Glu Arg Asp Glu Gln Gly Ser Lys Ile Asn
2225                2230                2235                2240

Asp Phe Phe Leu Arg Ser Glu Asp Leu Phe Asn Glu Met Asn Trp Gln
                2245                2250                2255
Lys Lys Leu Arg Ala Gln Pro Val Leu Tyr Trp Cys Ala Arg Asn Met
            2260                2265                2270
Ser Phe Trp Ser Ser Ile Ser Phe Asn Leu Ala Val Leu Met Asn Leu
        2275                2280                2285
Leu Val Ala Phe Phe Tyr Pro Phe Lys Gly Val Arg Gly Gly Thr Leu
    2290                2295                2300
Glu Pro His Trp Ser Gly Leu Leu Trp Thr Ala Met Leu Ile Ser Leu
2305                2310                2315                2320
Ala Ile Val Ile Ala Leu Pro Lys Pro His Gly Ile Arg Ala Leu Ile
                2325                2330                2335
Ala Ser Thr Ile Leu Arg Leu Ile Phe Ser Val Gly Leu Gln Pro Thr
            2340                2345                2350
Leu Phe Leu Leu Gly Ala Phe Asn Val Cys Asn Lys Ile Ile Phe Leu
        2355                2360                2365
Met Ser Phe Val Gly Asn Cys Gly Thr Phe Thr Arg Gly Tyr Arg Ala
    2370                2375                2380
Met Val Leu Asp Val Glu Phe Leu Tyr His Leu Leu Tyr Leu Leu Ile
2385                2390                2395                2400

Cys Ala Met Gly Leu Phe Val His Glu Phe Phe Tyr Ser Leu Leu Leu
                2405                2410                2415
Phe Asp Leu Val Tyr Arg Glu Glu Thr Leu Leu Asn Val Ile Lys Ser
            2420                2425                2430
Val Thr Arg Asn Gly Arg Ser Ile Ile Leu Thr Ala Val Leu Ala Leu
        2435                2440                2445
Ile Leu Val Tyr Leu Phe Ser Ile Val Gly Tyr Leu Phe Phe Lys Asp
    2450                2455                2460
Asp Phe Ile Leu Glu Val Asp Arg Leu Pro Asn Glu Thr Ala Val Pro
2465                2470                2475                2480
Glu Thr Gly Glu Ser Leu Ala Asn Asp Phe Leu Tyr Ser Asp Val Cys
                2485                2490                2495
Arg Val Glu Thr Gly Glu Asn Cys Thr Ser Pro Ala Pro Lys Glu Glu
            2500                2505                2510
Leu Leu Pro Ala Glu Glu Thr Glu Gln Asp Lys Glu His Thr Cys Glu
        2515                2520                2525
Thr Leu Leu Met Cys Ile Val Thr Val Leu Ser His Gly Leu Arg Ser
    2530                2535                2540
Gly Gly Gly Val Gly Asp Val Leu Arg Lys Pro Ser Lys Glu Glu Pro
2545                2550                2555                2560

Leu Phe Ala Ala Arg Val Ile Tyr Asp Leu Leu Phe Phe Phe Met Val
                2565                2570                2575

Ile Ile Ile Val Leu Asn Leu Ile Phe Gly Val Ile Ile Asp Thr Phe
            2580                2585                2590

Ala Asp Leu Arg Ser Glu Lys Gln Lys Lys Glu Glu Ile Leu Lys Thr
        2595                2600                2605

Thr Cys Phe Ile Cys Gly Leu Glu Arg Asp Lys Phe Asp Asn Lys Thr
    2610                2615                2620

Val Thr Phe Glu Glu His Ile Lys Glu Glu His Asn Met Trp His Tyr
2625                2630                2635                2640

Leu Cys Phe Ile Val Leu Val Lys Val Lys Asp Ser Thr Glu Tyr Thr
                2645                2650                2655

Gly Pro Glu Ser Tyr Val Ala Glu Met Ile Arg Glu Arg Asn Leu Asp
            2660                2665                2670

Trp Phe Leu Arg Met Arg Ala Met Ser Leu Val Ser Ser Asp Ser Glu
        2675                2680                2685

Gly Glu Gln Asn Glu Leu Arg Asn Leu Gln Glu Lys Leu Glu Ser Thr
    2690                2695                2700

Met Lys Leu Val Thr Asn Leu Ser Gly Gln Leu Ser Glu Leu Lys Asp
2705                2710                2715                2720

Gln Met Thr Glu Gln Arg Lys Gln Lys Gln Arg Ile Gly Leu Leu Gly
                2725                2730                2735
His Pro Pro His Met Asn Val Asn Pro Gln Gln Pro Ala
            2740                2745

## Description

### FIELD OF THE INVENTION

The present invention relates to a novel protein which binds to an IP₃ receptor. This protein is useful as an IP₃ indicator intracellularly and in a cell-free system.

### BACKGROUND OF THE INVENTION

The hydrolysis of phosphatidylinositol 4,5-bisphosphate in response to cell surface receptor activation leads to the production of an intracellular second messenger, inositol 1,4,5-trisphosphate (IP₃). IP₃ induces the release of Ca²⁺ from intracellular Ca²⁺ storage organelles, mainly the endoplasmic reticulum, by binding to the IP₃ receptor (IP₃R). In these IP₃/Ca²⁺ signalling cascades, the IP₃ receptor works as a signal converter to convert the IP₃ signal into the Ca²⁺ signal (1-3).

The IP₃ receptor is a tetrameric intracellular IP₃-gated Ca²⁺ release channel (3, 4). There are three distinct types of IP₃ receptor in mammals (5-7). The IP₃ receptor Type I (IP₃R1) is highly expressed in the central nervous system, particularly in the cerebellum (8, 9). Mouse IP₃R1 is composed of 2749 amino acids (5), and is divided into three functionally distinct regions: the IP₃-binding domain near the N terminus, the channel-forming domain with six membrane-spanning regions close to the C terminus, and the regulatory domain separating the two regions (10, 11). Deletion mutagenesis analysis of the IP₃-binding domain has shown that amino acid residues 226 to 578 of the IP₃ receptor are of the minimum area required for specific and high affinity ligand binding, thus assigned to the IP₃ binding core (12). The precise gating mechanism of the IP₃ receptor channel triggered by IP₃ remains unclear, but IP₃ binding induces an undefined conformational change in the IP₃ receptor, which may cause channel opening (10). Besides the channel opening, such IP₃-induced conformational change has been supposed to be responsible for degradation of the IP₃ receptor (13, 14).

The increase in the cytoplasmic Ca²⁺ concentration resulting from IP₃ receptor activation regulates the activities of a variety of downstream target molecules. These downstream target molecules play key roles in many aspects of cellular responses, including fertilization, development, proliferation, secretion and synaptic plasticity (1, 2, 15). To control such a vast array of cell functions, Ca²⁺ signals need to be precisely regulated in terms of space, time and amplitude (2, 15). Such a complex regulation of Ca²⁺ signals has been partly attributed to the diversity of IP₃ receptor isoform expression, assembly of heterotetrameric complexes of IP₃ receptor isoforms, subcellular distributions of IP₃ receptors, and regulation of IP₃ receptors by Ca²⁺ itself, ATP and phosphorylation (3, 4, 16). IP₃ receptor channels are also regulated by their interacting proteins (4, 17), including calmodulin (18, 19), FKBP12 (20-24), calcineurin (21, 23, 24, 25), ankyrin (26-28), the σ-1 receptor (28), chromogranin A and B (29-31), IRAG (32), Fyn (33), BANK (34) and the like. Moreover, a family termed CaBP has been shown to interact with the IP₃ receptor in a Ca²⁺-dependent manner, and to directly activate the IP₃ receptor in the absence of IP₃ (35). The IP₃ receptor has also been demonstrated to be physically coupled to its upstream or downstream signaling molecules by protein-protein interactions. For example, the IP₃ receptor is coupled with group 1 metabotropic glutamate receptors (mGluRs) via Homer family of proteins (36), and with B₂ bradykinin receptors (B₂Rs) by unknown mechanism (37). Activation of mGluRs and B₂Rs lead to the production of IP₃ in close proximity to the IP₃ receptor, resulting in efficient and specific signal propagation.

As described above, the IP₃ molecule is an important second messenger that controls a variety of cell functions, and changes in its intracellular concentration may be under temporal and spatial control. To date, a method using [³H]IP₃ as an indicator effective for quantifying IP₃ in vitro has been developed, but such a method is not simple because of, for example, the need of radioactive isotopes. Further, unlike the case of other second messengers such as Ca²⁺ and cAMP, the existing methods for sequentially detecting changes in IP₃ concentration within a living cell all involve monitoring transfer from the cell membrane of GFP-pleckstrin homology domain to cytoplasm. This method is not necessarily effective, because it is indirect and has problems in terms of quantitative ability, spatial resolution and the like. These are obstructions to intracellular IP₃ dynamics analysis.

### SUMMARY OF THE INVENTION

The present invention provides a novel IP₃ receptor-binding protein that can be an effective indicator for the detection and quantification of IP₃, and a method for detecting and/or quantifying IP₃ using the protein. In addition, this protein works as a messenger molecule after it is released from IP₃ receptor with IP₃ and this protein binds to Na, bicarbonate co-transporter and PIP kinase to produce PIP₂. Therefore, it works to modulate the function of PIP₂ and Na⁺ and bicarbonate ions. Since this protein binds to the same site as IP₃, and competes with IP₃, this protein can be used as a modulator of IP₃ induced Ca²⁺ release.

To address the above problems, we have noticed and eagerly studied IP₃ receptor-binding proteins, particularly a molecule which interacts with the IP₃ receptor and whose interaction with the IP₃ receptor is controlled by IP₃. Using an affinity column, to which a protein of the N-terminal 2217 amino acid residues corresponding to the large portion of the cytoplasm region of IP₃R1 (the amino acid sequence is represented by SEQ ID NO: 7) are bound, and eluting a protein bound to the column with IP₃, we have identified a novel IP₃ receptor-binding protein, and named it IRBIT (IP₃R-binding protein released with inositol 1,4,5-trisphosphate). IRBIT binds to IP₃R1 in vitro and in vivo, and the subcellular localization agrees well with that of IP₃R1. Further, it was also revealed that IRBIT is dissociated from IP₃R1 by IP₃ at a physiological concentration. Based on these results, we have considered that IRBIT is useful as an IP₃ indicator, modulator of IP₃-induced Ca²⁺ releasing activity, and modulator of PIP₂ function and Na²⁺ and bicarbonate ions, and thus completed the present invention.

The present invention relates to the following embodiments:
What is claimed is:
1. A protein (a), (b) or (c) as shown below:
   (a) a protein (IRBIT), comprising an amino acid sequence represented by SEQ ID NO: 1,
   (b) a protein, comprising an amino acid sequence of the 1-104 of SEQ ID NO: 1, and
   (c) a protein, comprising an amino acid sequence derived from the amino acid sequence of SEQ ID NO: 1 or an amino acid sequence of the 1-104 of SEQ ID NO: 1, which contains a deletion, substitution or addition of at least one of amino acid residues, and binding to an IP₃ receptor.
2. A gene, encoding the protein of claim 1.
3. A gene, comprising a DNA (a), (b) or (c) as shown below:
   (a) a DNA, comprising a nucleotide sequence of SEQ ID NO: 2,
   (b) a DNA, comprising a nucleotide sequence of the 1 to 312 of SEQ ID NO: 2, and
   (c) a DNA, hybridizing under stringent conditions to a DNA that is complementary to the DNA comprising the nucleotide sequence of (a) or (b), and encoding a protein that binds to the IP₃ receptor.
4. A vector, containing the gene of claim 2 or 3.
5. The vector of claim 4, wherein a promoter is operably linked to the gene of claim 2 or 3.
6. The vector of claim 5, wherein the gene of claim 2 or 3 is linked in-frame with a gene encoding a fluorescent protein or a photoprotein.
7. The vector of claim 6, wherein the fluourescent protein is selected from the group consisting of a green fluorescent protein (GFP), a cyan fluorescent protein (CFP), yellow fluorescent protein (YFP), Venus and FlAsH and the derivatives thereof.
8. A host cell, containing the vector of any one of claims 4 to 7.
9. The host cell of claim 8, which is a mammalian cell.
10. An IP₃ indicator for detecting IP₃ in a sample, containing the protein of claim 1.
11. The IP₃ indicator of claim 10, wherein the protein of claim 1 is fused to a fluorescent protein or a photoprotein.
12. The IP₃ indicator of claim 11, which is used for detecting IP₃ by the FRET method using a combination of the protein of claim 1 labeled with one fluorescent molecule of a combination of two fluorescent molecules applicable to the FRET method, and a protein containing at least the IP₃-binding domain of the IP₃ receptor labeled with the other fluorescent molecule of the above combination.
13. The IP₃ indicator of claim 12, wherein the combination of two fluorescent molecules is a combination of Venus and CFP, a combination of YFP and CFP, or a combination of FlAsH and CFP.
14. An antibody or a functional fragment thereof, specifically recognizing the protein of claim 1.
15. The antibody or the functional fragment thereof of claim 14, which is a rabbit polyclonal antibody or a fragment thereof.
16. A method for measuring IP₃ concentration in a sample, comprising:
   (i) preparing a protein complex comprising an IRBIT or a protein that contains the IP₃ receptor-binding domain of the IRBIT and a protein that contains at least the IP₃ binding domain of the IP₃ receptor,
   (ii) allowing the protein complex to contact with the sample, and
   (iii) quantifying a free IRBIT or a protein containing the IP₃ receptor-binding domain of the IRBIT and/or the complex.
17. The method of claim 16, wherein the quantification in (iii) above is performed utilizing an immunochemical reaction system using an anti-IRBIT antibody and/or an anti-IP₃ receptor antibody.
18. The method of claim 16 or 17, wherein the quantification in (iii) above is performed by previously labeling an IRBIT or a protein containing the IP₃ receptor-binding domain of the IRBIT or a protein containing at least the IP₃ binding domain of the IP₃ receptor, and then measuring signals originating from the labeled protein.
19. The method of claim 18, wherein the label is a fluorescent label.
20. The method of claim 16, wherein the quantification of (iii) above is performed by the FRET method.
21. The method of claim 20, which uses an IRBIT or a protein containing the IP₃ receptor-binding domain of the IRBIT labeled with YFP or Venus, and a protein containing at least the IP₃-binding domain of the IP₃ receptor labeled with CFP.
22. The method of claim 20, which uses an IRBIT or a protein containing the IP₃ receptor-binding domain of the IRBIT labeled with CFP, and a protein containing at least the IP₃-binding domain of the IP₃ receptor labeled with Venus or YFP.
23. A method for quantifying the intracellular IP₃ concentration of living cells, comprising:
   (i) introducing into a cell both of a gene encoding an IRBIT or a protein containing the IP₃ receptor-binding domain of the IRBIT, labeled with one fluorescent molecule of a combination of two fluorescent molecules applicable to the FRET method, and a gene encoding a protein containing at least the IP₃ binding domain of the IP₃ receptor labeled with the other fluorescent molecule of the above combination, such that the genes can be expressed, and
   (ii) irradiating the cells with the excitation wavelength of the fluorescent molecule on the lower wavelength side of the above two fluorescent molecules, and measuring under a fluorescence microscope the fluorescence wavelength of the molecule and the fluorescence wavelength of the other fluorescent molecule on the longer wavelength side.
24. The method of claim 23, wherein the combination comprising two fluorescent molecules applicable to the FRET method is a combination of Venus and CFP, a combination of YFP and CFP, or a combination of FlAsH and CFP.
25. A protein complex for quantifying IP₃ concentration, comprising an IRBIT or a protein containing the IP₃ receptor-binding domain of the IRBIT and a protein containing the IP₃ receptor or at least the IP₃ binding domain thereof.
26. A vector for quantifying intracellular IP₃ concentration of living cells, containing a gene encoding an IRBIT or a protein containing the IP₃ receptor-binding domain of the IRBIT, labeled with one fluorescent molecule of a combination of two fluorescent molecules applicable to the FRET method, and a gene encoding a protein containing at least the IP₃ binding domain of the IP₃ receptor labeled with the other fluorescent molecule of the above combination such that these genes can be expressed.
27. The vector of claim 26, wherein the combination comprising two fluorescent molecules is a combination of Venus and CFP, a combination of YFP and CFP, or a combination of FlAsH and CFP.

The present invention will be detailed described.

A novel IP₃ receptor protein, IRBIT, that we have newly cloned comprises the amino acid sequence represented by SEQ ID NO: 1. The IRBIT is composed of two reagions, the N-terminal region (amino acids 1-104) and the C-terminal region (amino acids 105-530). The C-terminal region is homologous to S-adenosylhomocysteine hydrolase but does not show enzyme activity, and the N-terminal region is an essential region for binding with the IP₃ receptor (in this specification, the IP₃ receptor is referred to as any subtype of the IP₃ receptor including IP₃R1 and IP₃ receptor type II (IP₃R2)). The characteristics of the protein are as follows:
(1) the protein is a neutral protein (calculated pI of 6.48), but the N-terminal region is relatively acidic (calculated pI of 4.98);
(2) seven potential phosphorylation sites are concentrated in the N-terminal region, so that phosphorylation is indicated to be necessary for the interaction with IP₃R1;
(3) the 508 lysine residue of IP₃R1 is essential for binding to IP₃ is also essential for binding to IRBIT;
(4) the interaction of IRBIT with IP₃R1 is disrupted by IP₃; and
(5) based on the facts that the interaction with IP₃R1 is dissociated by a high salt buffer and the protein is extracted from a crude microsome fraction, the interaction of IRBIT with IP₃R1 is dependent on an electrostatic bond.

Therefore, the present invention encompasses IRBIT protein, the N-terminal region (amino acids 1-104) that is essential for binding of IRBIT to the IP₃ receptor, and proteins containing these amino acid sequences. In some cases, a protein containing the 1-277 amino acid sequence of IRBIT is preferred. The present invention also encompasses a protein, which is a mutant of these proteins that maintain IP₃ receptor-binding activity, and comprises an amino acid sequence derived from the amino acid sequence of IRBIT , which contains a deletion, substitution or addition of at least one of amino acids, preferably 1 to 50, more preferably 1 to 20, further preferably 1 to 10, and particularly preferably 1 to 5 amino acid(s).

The present invention further encompasses genes encoding these proteins. The gene is, for example, DNA comprising the nucleotide sequence represented by SEQ ID NO: 2 encoding IRBIT protein, or comprising a nucleotide sequence of the 1-312 nucleotides of SEQ ID NO: 2 encoding the N-terminal region (amino acids 1-104) that is the IP₃ receptor-binding domain of IRBIT. The present invention also includes these DNAs or DNAs hybridizing under stringent conditions to complementary strands of the DNAs. Here, the stringent conditions are referred to as, for example, conditions with a sodium concentration of 10 mM to 300 mM, and preferably 37 to 65°C, and more preferably 42°C. Alternatively, such conditions can be achieved using ECL™ direct nucleic acid labeling and detection system (Amersham Pharmacia) according to the description of the instructions included with the system. The present invention also includes a DNA comprising a nucleotide sequence derived from the nucleotide sequence of SEQ ID NO: 2 or the nucleotide sequence of the 1-312 of SEQ ID NO: 2, which contains a deletion, substitution or addition of at least one of nucleotides, and encoding a protein having IP₃ receptor-binding activity. Here, the number of nucleotides to be deleted, substituted or added is not specifically limited, and is preferably 1 to 100, more preferably 1 to 50, further more preferably 1 to 20, and most preferably 1 to 10. The nucleotide sequence of such a nucleic acid has homology to the nucleotide sequence represented by SEQ ID NO: 1 when calculated using BLAST or the like (for example, when the default of BLAST, specifically the parameters of initial conditions are used) of 70% or more, preferably 90% or more, further more preferably 95% or more, 96% or more, 97% or more, 98% or more or 99% or more. The present invention further encompasses RNAs corresponding to these DNAs, a vector containing these DNAs, a vector wherein a promoter is operably linked to the DNA so as to enable the expression of the above DNA, a vector wherein a DNA encoding any label for labeling the expression product (IRBIT molecule or the like) of the above DNA that is ligated in-frame to the DNA, and a host cell containing the vector (insect cells, *Escherichia coli* and mammalian cells are preferred, but examples are not limited thereto). Examples of the above labels may be any known labels and include, but are not limited to, labels for utilizing immunological reaction or protein-protein binding such as a histidine tag (His-tag), glutathioneS-transferase (GST) and biotin, as well as fluorescent proteins or photoproteins such as green fluorescent protein (GFP), CFP, Venus, FlAsH, ReAsH and derivatives thereof.

Further, IRBIT is a protein that can be dissociated from the IP₃ receptor by IP₃ selectively in a concentration-dependent manner. For example, when the IP₃ receptor is the IP₃ receptor type I, the EC50 value for the above dissociation is approximately 0.5 µM. This is higher than a Kd value (Kd=83 to 100 nM: see 60, 61) during the binding of IP₃R1 to IP₃ measured by IP₃ binding assay according to a conventional method. However, the difference may be due to a difference in buffer conditions (this assay has been performed under conditions ranging from pH 8.0 to 8.3 and with low ion intensity) based on the fact that the binding affinity of IP₃ with the IP₃ receptor is largely dependent on pH and ion intensity (62-64). Studies according to a method using a surface plasmon resonance biosensor, which is different from a conventional method, showed a Kd value of 336 nM, when the N-terminal region of IP₃R1 (amino acids 1-604) was measured under physiological conditions (pH 7.4 and 150 mM NaCl) (64), suggesting that the affinity in this case was approximately 7.5-fold lower than the Kd value determined by a conventional assay. This value is also close to the EC50 of IRBIT (approximately 0.5 µM: measured under conditions of pH 7.4 and 100 mM NaCl) required for its dissociation from the interaction with IP₃R1 (In the present invention, GST fusion protein was used for the convenience of the experiment, and this protein is referred to as GST-EL in this specification). Taken together with these findings, it can be considered that IRBIT is dissociated from the IP₃ receptor by binding of IP₃ to the IP₃ receptor. This is supported by data, such as the results of measurement made by Luzzi et al. using a detector cell/capillary electrophoresis system, wherein the intracellular IP₃ concentration was tens of nM before stimulation, and then increased to few µM after stimulation (50). As described above, the EC50 of IP₃ required for the dissociation of IRBIT from IP₃R1 is approximately 0.5 µM, and this value is within the fluctuation range of the intracellular IP₃ concentration before and after stimulation. Thus, it is strongly suggested that IRBIT is dissociated from the IP₃ receptor following IP₃ production induced by extracellular stimulation.

With the characteristic of IRBIT that it is dissociated from the IP₃ receptor by IP₃ in a concentration-dependent manner, a protein containing at least IRBIT or its IP₃ receptor-binding domain can be used as a novel IP₃ indicator. Specifically, for example, using IRBIT, a sample is allowed to contact with an IRBIT-IP₃ receptor complex (the IP₃ receptor in this case is not necessarily a complete molecule, but may be a fragment containing at least a region to which both IP₃ and IRBIT can bind (hereinafter, referred to as IP₃BD)). Then, the amount of IRBIT that has been dissociated from the IP₃ receptor is quantified, or conversely the amount of IRBIT-IP₃ receptor/IP₃BD complex remaining after contact with IP₃ is quantified, so as to make it possible to quantitatively detecting IP₃. The same result is obtained when a protein containing at least the IP₃ receptor-binding domain of IRBIT is used.

Quantitative detection of free IRBIT or an IRBIT-IP₃ receptor complex can also be performed by immunological techniques.

The term "antibody" in the present specification includes complete antibody molecules, fragments retaining antigen-binding ability derived from the complete antibody, and derivatives thereof. Antibodies against IRBIT may be monoclonal or polyclonal antibodies, both of which can be obtained by any general method that involves immunizing animals with an IRBIT molecule or a peptide (a part of the IRBIT molecule). A purification method of these antibody molecules and a purification method which involves cleaving into antibody fragments having binding affinity for IRBIT are also known by persons skilled in the art. Any appropriate method can be selected from these methods, as desired. Persons skilled in the art can readily detect free IRBIT using these antibody molecules. Persons skilled in the art can also readily perform a method for detecting an IRBIT-IP₃ receptor/IP₃BD complex using the antibody molecules. For example, IP₃R1 is previously bound to a solid phase support (beads, assay plate surfaces or the like), and then IRBIT bound via IP₃R1 to the solid phase can be detected.

Further, IRBIT is labeled with an appropriate molecule, and then the labels are detected, so that free IRBIT or an IRBIT-IP₃ receptor/IP₃BD complex can be quantitatively detected. Examples of known molecules for labeling proteins include radioactive labels, immuno-labels, dye labels, fluorescent labels and luminescent labels. Labeling can be performed by binding any one of these labeling molecules to an IRBIT molecule by any known method according to the labeling molecule. Persons skilled in the art can also readily detect signals derived from the labels used herein based on the known art.

First, a complex (referred to as an IRBIT-IP₃ receptor/IP₃BD complex) comprising a protein that contains at least IRBIT or the IP₃ receptor-binding domain thereof and the IP₃ receptor or IP₃BD is prepared. Since IRBIT forms a complex with the IP₃ receptor intracellularly, it can also be purified from natural cells (cerebellum neurons and the like) expressing the IP₃ receptor. Alternatively, from cells (*Escherichia coli,* Sf9 insect cells or the like) forced to express an IRBIT or a protein containing at least the IP₃ receptor-binding domain of the IRBIT and a protein containing IP₃ receptor molecules or IP₃BD, a microsome fraction can be fractionated by a biochemical fractionation method. Further alternatively, after forced expression of the above protein having molecular labels (histidine tag and the like) attached thereto, the protein can be extracted using the labels from a cell lysate. In some cases, other known purification methods (column chromatography and the like) may also be used.

Moreover, IRBIT and IP₃PD are separately prepared, and then a complex thereof can be formed. Specifically, for example, GST-IP₃BD expressed in *Escherichia coli* is purified with glutathione-Sepharose, and then a protein containing IRBIT or its IP₃ receptor-binding region is expressed in *Escherichia coli* or Sf9 cells. Proteins containing the IRBIT or its IP₃ receptor-binding region are allowed to contact with GST-IP₃BD to form complexes, followed by glutathione-Sepharose pull-down, so that the complexes can be purified. Further alternatively, the above complex may also be prepared by pulling down IRBIT in the tissue with GST-IP₃BD using glutathione-Sepharose from, for example, a high salt extract from the cerebellum.

A sample containing IP₃ is allowed to contact with the thus prepared IRBIT-IP₃ receptor/IP₃BD complex (prepared, for example, at approximately 1 to 100 µM in 10 mM Hepes, 100 mM NaCl, 2 mM EDTA and 1 mM 2-ME (pH7.4)), followed by incubation on ice for approximately 5 to 30 minutes. Then, free IRBIT or the remaining IRBIT-IP₃ receptor/IP₃BD complex present in the solution is detected. The IRBIT-IP₃ receptor/IP₃BD complex to be used in reaction is previously bound to solid phase carriers, and then the solid phase carriers are separated from the liquid phase after reaction, so that free IRBIT in the liquid phase can be detected, or IRBIT in the IRBIT-IP₃ receptor/IP₃BD complex bound to the solid phase carriers can be detected. IRBIT may be detected with an immunological reaction system using an antibody that recognizes IRBIT. When labeled IRBIT is used, IRBIT can also be detected by detecting the label.

Furthermore, in an embodiment of the present invention, IP₃ can also be quantified by a method using fluorescence resonance energy transfer, FRET.

Fluorescence resonance energy transfer (FRET) is a phenomenon wherein when two fluorephores are in close proximity and in the right orientation, excitation energy transfers from one fluorophore (energy donor) to the other fluorophore (energy acceptor) on the longer wavelength side. By labeling two molecules with certain 2 types of fluorescent molecules, changes in the interaction between the two molecules can be measured as changes in FRET efficiency. Further, changes in the concentration of another molecule that regulates the interaction between molecules can be measured.

Based on the FRET method, a method (72) for detecting changes in intracellular Ca²⁺ concentration, a method (71) for detecting changes in cAMP concentration, and the like have been developed.

IRBIT binds to the IP₃BD of the IP₃ receptor, and is dissociated from the IP₃ receptor by IP₃. Using this characteristic, IRBIT and IP₃BD are labeled respectively with a combination of 2 types of fluorescent labels that are applicable to FRET. Thus, changes in in vitro or intracellular IP₃ concentration can be detected. Specifically, for example, IRBIT is labeled with a yellow fluorescent protein (YFP) or with the improved protein thereof, Venus, and IP₃BD is labeled with cyan fluorescent protein (CFP). In the absence of IP₃, IRBIT binds to IP₃BD and FRET occurs from CFP to Venus. However, in the presence of IP₃, the two are dissociated from each other, and no FRET occurs. Further, when Venus-IRBIT and CFP-IP₃BD are bound via a linker sequence (Venus-IRBIT-IP₃BD-CFP), the molar ratios of the two will be the same, and this is efficient. When IP₃ concentration is measured in vitro, Venus-IRBIT-IP₃BD-CFP protein is added to a cell extract or the like, and then FRET is measured. Further, introduction of a Venus-IRBIT-IP₃BD-CFP gene into a cell enables temporal and spatial analysis of changes in IP₃ concentration within living cells. Measurement with the FRET method can be performed using a fluorescent microscope, fluorometer or the like.

Examples of IP₃ detection methods using the FRET method will be described in more detail as follows.

First, a Venus-IRBIT-IP₃BD-CFP expression vector is prepared. Four cDNAs are amplified by PCR, each of which encodes a full-length IRBIT or a deletion mutant thereof containing a region sufficient for binding with IP₃BD (e.g., a mutant comprising amino acid sequence 1-104 or amino acid sequence 1-277), a protein containing the IP₃ binding region of IP₃ receptor type I (a region comprising amino acid sequence 224-604 of SEQ ID NO: 7), Venus, and CFP. Then these 4 cDNAs are inserted into a mammalian cell expression vector (e.g., pcDNA3) so as to match the reading frames. The order for insertion is not specifically limited. For example, the order of Venus-IRBIT-IP₃BD-CFP, wherein IRBIT and IP₃BD are placed inside and Venus and CFP are placed outside, can be employed. For preparation of a recombinant protein, the cDNAs are inserted into an *Escherichia coli* expression vector (e.g., pET) or Sf9 expression vector (e.g., pFastBac). In this case, a tag (e.g., a His tag) can be linked to the N-terminus or the C-terminus for purification.

Venus-IRBIT-IP₃BD-CFP is expressed in *Escherichia coli* or Sf9 cells, and is purified with ProBond resin (Invitrogen) or the like when a His tag is added. In some cases, it may be purified with an ion exchange column or a gel filtration column.

An IP₃ solution having a known concentration is added to the purified Venus-IRBIT-IP₃BD-CFP protein. The excitation wavelength (at around 440 nm) of CFP is applied to the solution, and then the CFP fluorescent wavelength (at around 480 nm, CFP fluorescence) and Venus fluorescent wavelength (at around 535 nm, FRET fluorescence) are measured. A calibration curve is then determined by plotting IP₃ concentrations versus FRET fluorescence-to-CFP fluorescence ratios. Venus-IRBIT-IP₃BD-CFP protein is added to a sample (e.g., a cell extract solution) with an unknown IP₃ concentration to find a FRET fluorescence-to-CFP fluorescence ratio, and then the ratio is applied to the calibration curve, thereby quantifying IP₃ concentration.

The IP₃ concentration in a living cell can also be measured by the FRET method. First, a pcDNA-Venus-IRBIT-IP₃BD-CFP gene is transfected into a culture cell (e.g., Cos-7 and HeLa) using a transfection reagent (e.g., TransIT (Mirus)). On 1-3 days after the transfection, cells on the glass bottom dish are observed using an inverted fluorescence microscope equipped with a cool CCD camera (e.g., IX71 (Olympus)). The CFP excitation wavelength (at around 440 nm) is applied, and then the CFP fluorescent wavelength (at around 480 nm, CFP fluorescence) and Venus fluorescent wavelength (at around 535 nm, FRET fluorescence) are measured. The FRET efficiency is quantified by taking the ratio of the two fluorescence intensities. An IP₃-producing agonist (ATP in the case of Cos-7, histamine in the case of HeLa, and the like) is added to the glass-bottom dish, and then the FRET efficiency is measured with time. When IP₃ is produced by the agonist, the FRET-to-CFP ratio is expected to be lowered.

The use of a combination of fluorescent labels, Venus and CFP, is as exemplified above. A combination of YFP and CFP or FlAsH and CFP can also be used, and use is not limited thereto. Further, various fluorescent molecules to be used for the FRET method are expected to be developed in the future, and they can also be used for implementing the present invention.

The present invention also encompasses the IP₃ indicator of the present invention or a kit for IP₃ detection to be used for the IP₃ detection method of the present invention. The indicator or the kit contains a protein containing at least the IP₃ receptor-binding region of IRBIT or a DNA or an RNA encoding the protein. In some cases, the indicator or the kit contains a protein containing at least the IP₃-binding region of IP₃ receptor, or a DNA or an RNA encoding the protein. Further, the indicator or the kit may also contain a labeling reagent (e.g., a fluorescent labeling compound) and/or an antibody that recognizes the above protein.

This specification includes part or all of the contents disclosed in specification and/or drawings of Japanese Patent Application No. 2002-299429, which are priority documents of present application.

### BRIEF DESCRIPTION OF THE DRAWINGS

FIG. 1 shows the purification of IRBIT and the cDNA cloning.

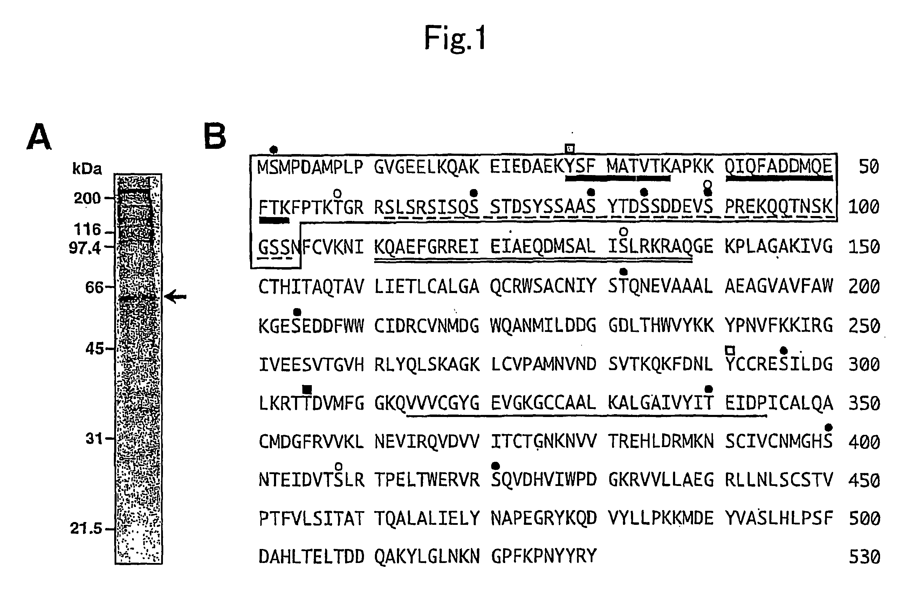

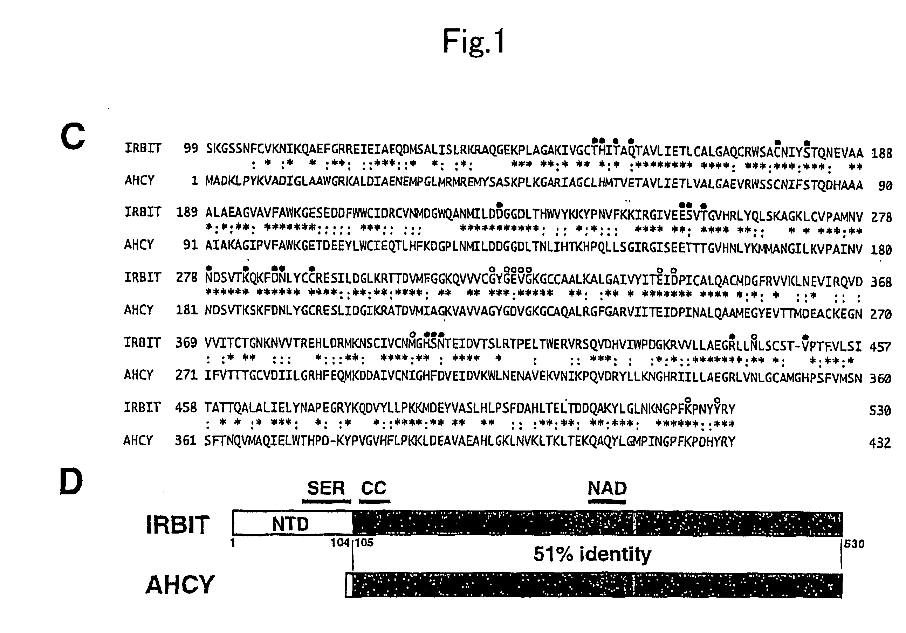

   FIG. 1A shows the result of SDS-PAGE of the IRBIT protein purified from the high salt extract from crude microsome of a rat cerebellum using the GST-EL column (IRBIT position is indicated by an arrow). IRBIT was eluted with 50 µM IP₃. The eluate was concentrated, separated by SDS-PAGE on 10% gel and stained with Coomassie Brilliant Blue. The bands observed at and above 200 kDa and at 100 to 200 kDa may be GST-EL slightly dissociated from resin and eluted and its degradates, respectively, since these bands were recognized by various types of anti-IP₃R1 antibodies. FIG1B shows the deduced amino acid sequences of IRBIT. Two portions resulted by digestion of purified IRBIT are bold-underlined. The serine-rich region is dashed-underlined. The coiled-coil region is double-underlined. The NAD⁺ binding site is underlined with a thin line. Putative phosphorylation sites for casein kinase, protein kinase C, protein kinase A/protein kinase G and tyrosine kinases are respectively indicated with closed circles, open circles, closed squares and open squares above the sequences. The N-terminal region is boxed with a solid line. FIG. 1C shows the sequence alignment of the C-terminal region of IRBIT and S-adenosylhomocysteine hydrolase (AHCY) (48). Identical residues are indicated with "*", and similar residues are indicated with ":". Residues involved in substrate binding and residues involved in NAD+ binding of S-adenosylhomocysteine hydrolase are indicated by closed circles and open circles, respectively. FIG. 1D is a schematic representation of the structure of IRBIT. NTR indicates the N-terminal region, CTR indicates the C-terminal region. SER indicates a serine-rich region, CC indicates a coiled-coil region, and NAD indicates an NAD⁺ binding site.
FIG. 2 shows that IRBIT had no S-adenosylhomocysteine hydrolase activity.
   The S-adenosylhomocysteine hydrolase activity in the hydrolytic direction of recombinant IRBIT-His (open circle), GST-IRBIT (triangle), GST (square), and S-adenosylhomocysteine hydrolase (closed circle) were measured according to the known method (43). Results are shown as the mean ± standard deviation from three independent experimental results.

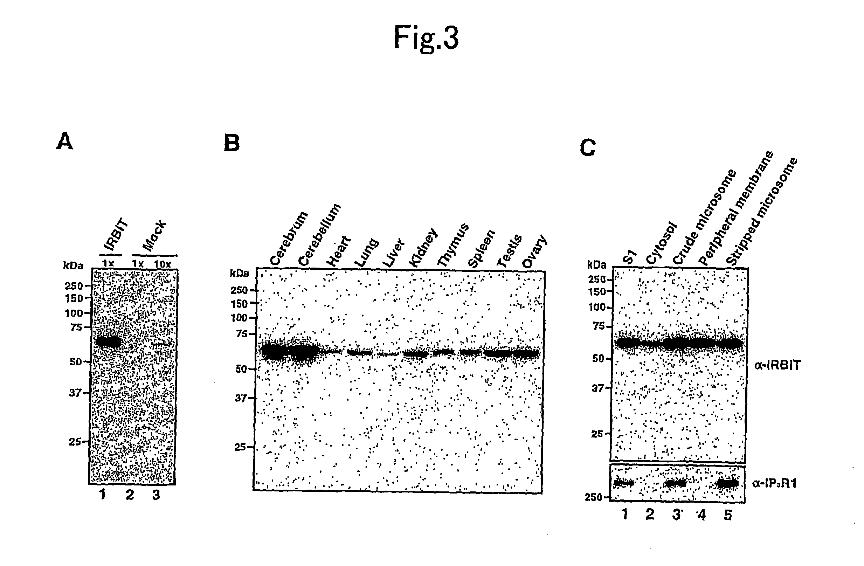

   FIG. 3 shows the tissue distribution and subcellular fractionation of IRBIT. FIG. 3A shows the results of western blot analysis of exogenously expressed IRBIT and endogenous IRBIT. The cell lysates of Cos-7 cells into which IRBIT (lane 1) and mock control (lanes 2 and 3) had been transfected were analyzed by Western blotting with anti-IRBIT antibody. In lane 3, a quantity of lysates 10 times greater than those in lanes 1 and 2 was loaded. FIG. 3B shows the results of examining the tissue distribution of IRBIT by analyzing S1 fractions (2 µg of protein) of mouse tissue by Western blotting using anti-IRBIT antibody. FIG. 3C shows the results of examining the subcellular fractionation of mouse cerebellum. S1 fraction of mouse tissue was centrifuged at 100,000 × g to obtain a cytosolic fraction (lane 2) and a crude microsome (lane 3). The crude microsome was extracted with a high salt buffer containing 500 mM NaCl and centrifuged at 100,000 x g to obtain a membrane-bound fraction (lane 4) and a stripped-crude microsome (lane 5). The upper panel in the figure shows the results of analyzing each fraction (1 µg of protein) by Western blotting using anti-IRBIT antibody, and the lower panel in the figure shows the results of analyzing by Western blotting using anti-IP₃R1 antibody.

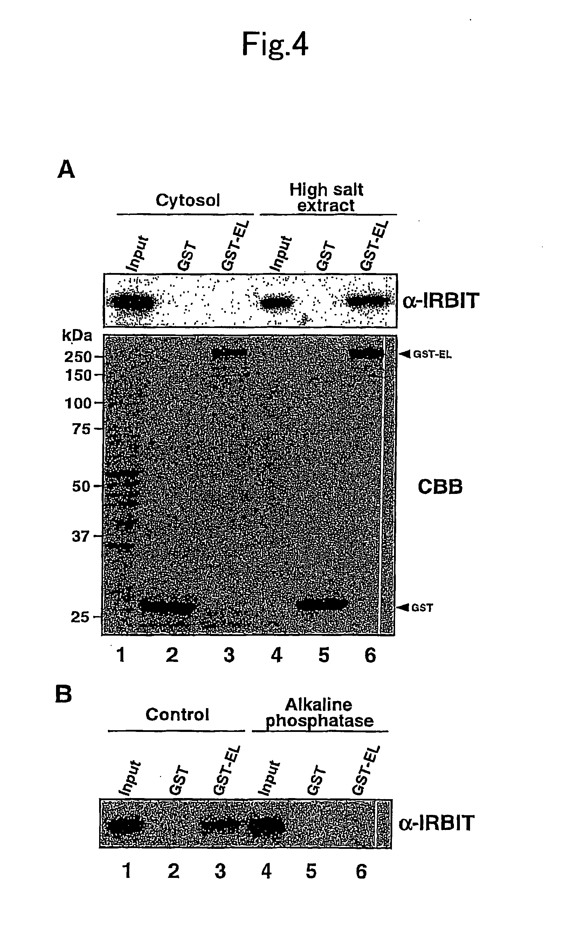

FIG. 4 shows that IRBIT in a high salt extract interacted with IP₃R1 in vitro, but IRBIT in the cytosol did not interact with IP₃R1. FIG. 4A shows the results of Western blotting analysis using anti-IRBIT antibody (upper panel) on samples prepared by incubating the mouse cerebellar cytosolic fraction (lanes 1 to 3) and the high salt extract (lanes 4 to 6) from the crude microsome with GST-EL (lanes 3 and 6) or GST (lanes 2 and 5), pulling down bound proteins with glutathione-Sepharose, and eluting with glutathione. GST-EL and GST pulled down by glutathione-Sepharose were stained with Coomassie Brilliant Blue (lower panel). FIG. 4B shows the results of analyzing IRBIT binding as shown in FIG. 4A by treating the high salt extract of crude microsome obtained from mouse cerebella without (lanes 1 to 3) or with (lanes 4 to 6) alkaline phosphatase, and then incubating with GST-EL (lanes 3 and 6) or GST (lanes 2 and 5).

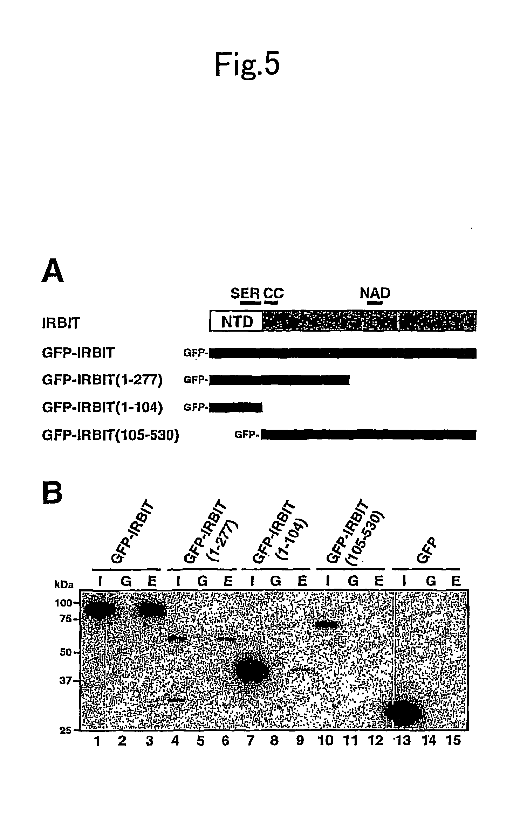

FIG. 5 shows that the N-terminal region of IRBIT was essential for the interaction with IP₃R1. FIG. 5A is a schematic representation of the structure of IRBIT and its GFP-tagged truncated mutants. In FIG. 5B, the lysates of Cos-7 cells expressing GFP-IRBIT (lanes 1 to 3), GFP-IRBIT (1-277) (lanes 4 to 6), GFP-IRBIT (1-104) (lanes 7 to 9), GFP-IRBIT (105-530) (lanes 10 to 12) and GFP (lanes 13-15) were used for GST pulldown assay. The lysates of Cos-7 cells expressing each GFP fusion protein (input; I) were incubated with GST-EL (E) or GST (G). Bound proteins were pulled down with glutathione-Sepharose, eluted with glutathione, and subjected to immunoblot analysis with anti-GFP antibody.

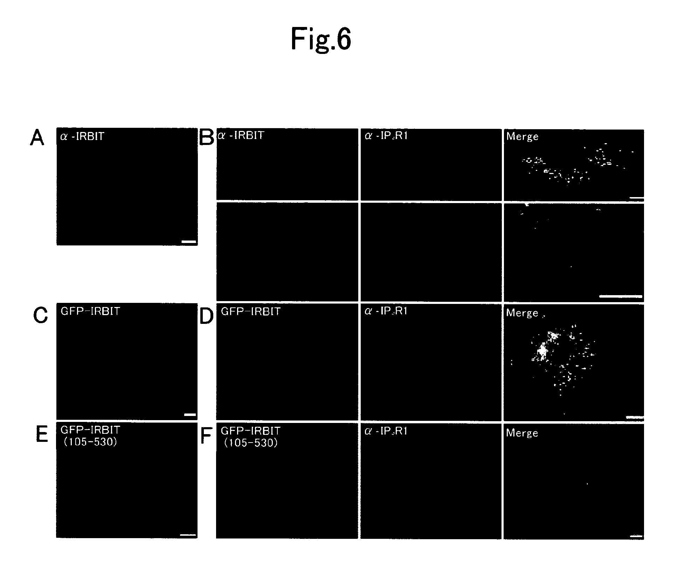

FIG. 6 shows that IRBIT co-localized with IP₃R1 in Cos-7 cells showing co-expression. IP₃R1 was transiently co-expressed in Cos-7 cells with IRBIT (A and B), GFP-IRBIT (C and D), and GFP-IRBIT (105-530) (E and F). The subcellular localization of the corresponding proteins was analyzed by indirect immunofluourescence (using anti-IP₃R1 and anti-IRBIT antibody) and fluorescence confocal microscopy (GFP-IRBIT and GFP-IRBIT (105-530)). B, D and F were permeabilized in saponin and cytosolic proteins were washed out prior to fixation of the cells. The left panels show IRBIT (B), GFP-IRBIT (D), and GFP-IRBIT (105-530) (F). The middle panels show IP₃R1. The right panels show the merged images of the left and middle panels. The lower panels (B) are higher-magnification images of upper panels (scale: 10 µm).

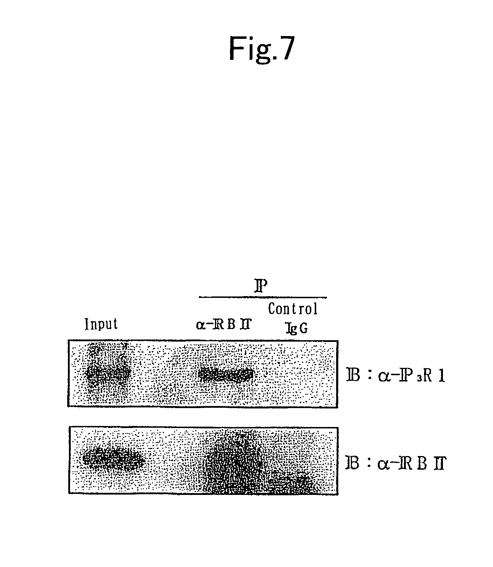

FIG. 7 shows IRBIT associated with IP₃R1 in vivo. The detergent extract of mouse cerebellar crude microsome was immunoprecipitated with anti-IRBIT antibody or control antibody. The immunoprecipitates were subjected to SDS-PAGE followed by Western blotting with anti-IP₃R1 antibody (upper panel) or anti-IRBIT (lower panel) antibody.

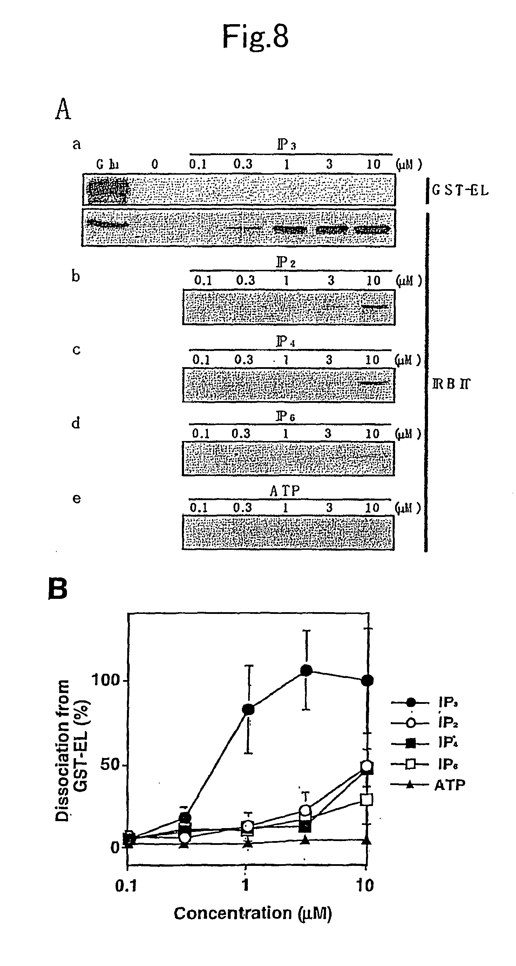

FIG. 8 shows that the physiological concentration of IP₃ selectively dissociated IRBIT from IP₃R1. FIG. 8A shows the incubation of the high salt extract of crude microsome obtained from mouse cerebella with GST-EL. Bound proteins were pulled down with glutathione-Sepharose, and eluted with glutathione (Glu) (a) or 0.1 to 10 µM IP₃ (a), IP₂ (b), IP₄ (c), IP₆ (d), or ATP (e). IRBIT in the extract was analyzed with anti-IRBIT antibody and Alexa 680-conjugated secondary antibody ("a" (lower panel) and panels "b" to "e"). GST-EL in the glutathione and 0.1 to 10 µM IP₃ eluate was analyzed with anti-GST antibody (a, upper panel). FIG. 8B shows the results of quantifying by infrared imaging system the signal intensity derived from IRBIT bands detected with anti-IRBIT antibody. The relative intensity was plotted against the concentration of an eluant. Results are shown as the mean ± standard deviation from at least three independent experiments.

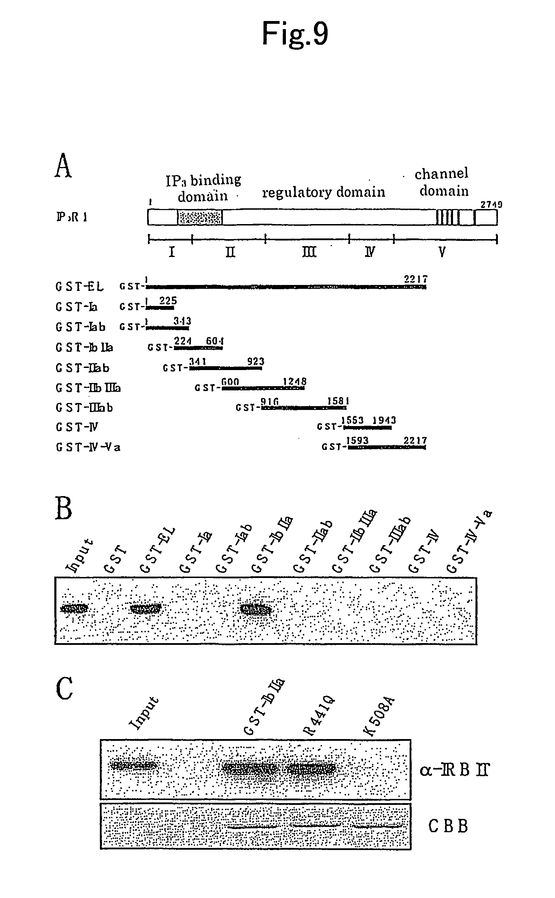

FIG. 9 shows that IRBIT interacted with the IP₃-binding region of IP₃R1 and Lys-508 of IP₃R1 was critical for the interaction. FIG. 9A is the schematic representation of the structure of mouse IP₃R1 and the recombinant GST fusion proteins used in this study. The IP₃ binding core region is indicated with a gray box. Putative membrane-spanning regions are indicated with solid vertical bars. Roman numbers I to V indicate the domain structure of IP₃R1 determined by the limited trypsin digestion (51). Numbers above the bars represent corresponding amino acid residue numbers. FIG. 9B shows the results of examining the IRBIT binding region of IP₃R1. The high salt extract of crude microsome obtained from mouse cerebella was incubated with GST fusion proteins shown in (A). Bound proteins were pulled down with glutathione-Sepharose, eluted with glutathione, and analyzed by Western blotting using anti-IRBIT antibody. FIG. 9C shows the results of performing pulldown assay for the high salt extract with GST-IbIIa, R441 Q and K508A, as in (B). Bound proteins were pulled down with glutathione-Sepharose, eluted with glutathione, and then analyzed by Western blotting using anti-IRBIT antibody (upper panel). GST fusion proteins pulled down with glutathione-Sepharose were stained with Coomassie Brilliant Blue (lower panel).

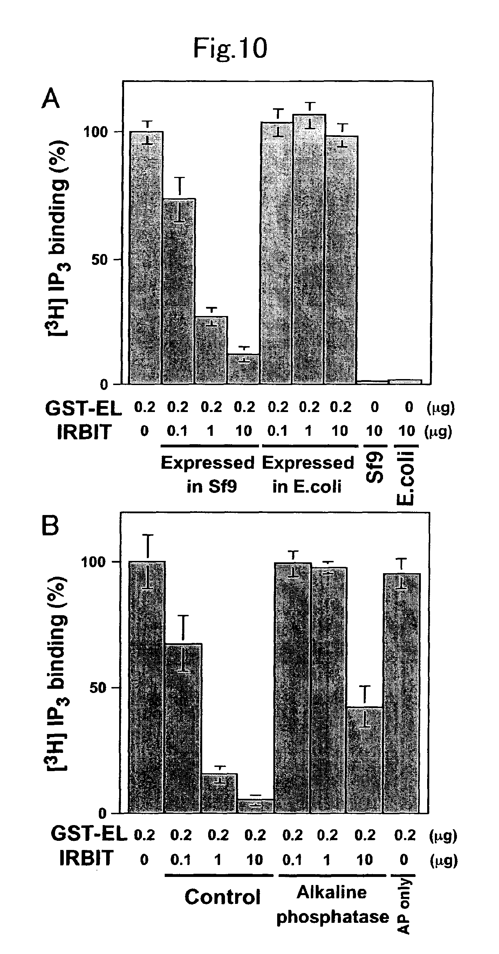

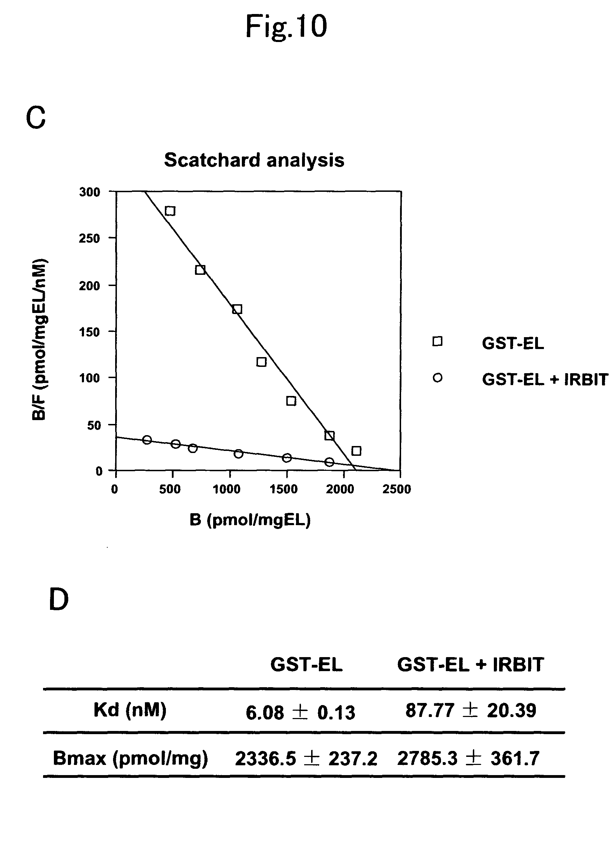

FIG. 10 shows that IRBIT lowered the affinity of IP₃R to IP₃ in a phosphorylation dependent manner. (A) 0.2 µg of GST-EL was incubated with 0.1, 1, or 10 µg of purified His-tagged IRBIT expressed in Sf9 cells or *E. coli* in a solution containing 50 mM Tris-HCl (pH 8.0), 1 mM EDTA, and 1 mM 2-mercaptoethanol, for 30 min on ice. Then 8.7 nM [³H]IP₃ (PerkinElmer Life Sciences) was added to the samples and incubated for 10 min on ice (total volume was 50 µl). The samples were mixed with 2 µl of 50 mg/ml γ-globulin and 50 µl of 30 % PEG6000, 50 mM Tris-HCl (pH 8.0), and incubated for 5 min on ice. Non-specific binding was measured in the presence of 10 µM cold IP₃. After centrifugation at 20,000 x g for 5 min, the precipitate was dissolved in SOLVABLE™ (Packard) and the radioactivity was measured with a liquid scintillation counter (Beckman Coulter). Results are shown as the mean ± S.D. from three independent experiments. (B) Purified His-IRBIT expressed in Sf9 cells was incubated with alkaline phosphatase for 30 min at 37 °C prior to the IP₃ binding assay described as in (A). (C and D) Scatchard analysis. 0.1 µg of GST-EL was incubated with or without 1 µg of purified His-tagged IRBIT expressed in Sf9 cells as described in (A). Then 2.17 nM [³H]IP₃ and 2-500 nM cold IP₃ were added to the samples and incubated for 10 min on ice (total volume was 100 µl). Non-specific binding was measured in the presence of 10 µM cold IP₃. [³H]IP₃ binding to GST-EL was measured as described in (A).

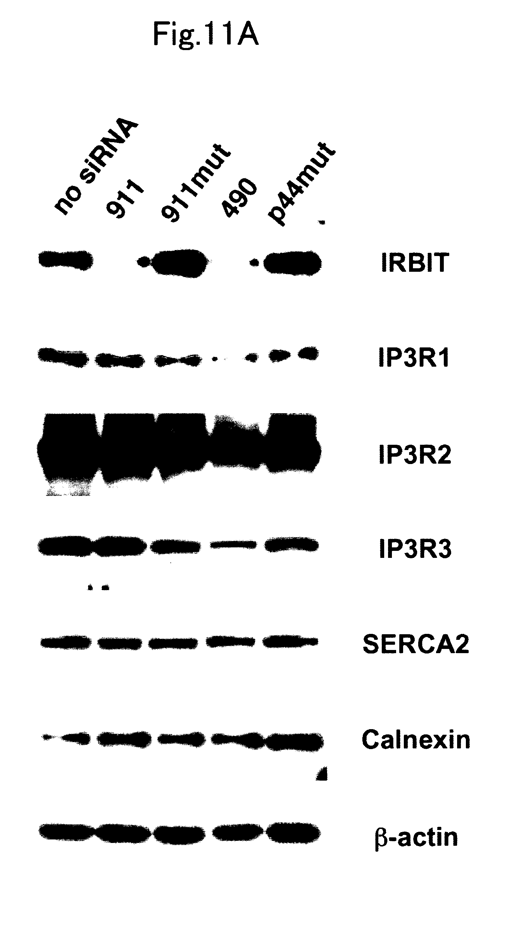

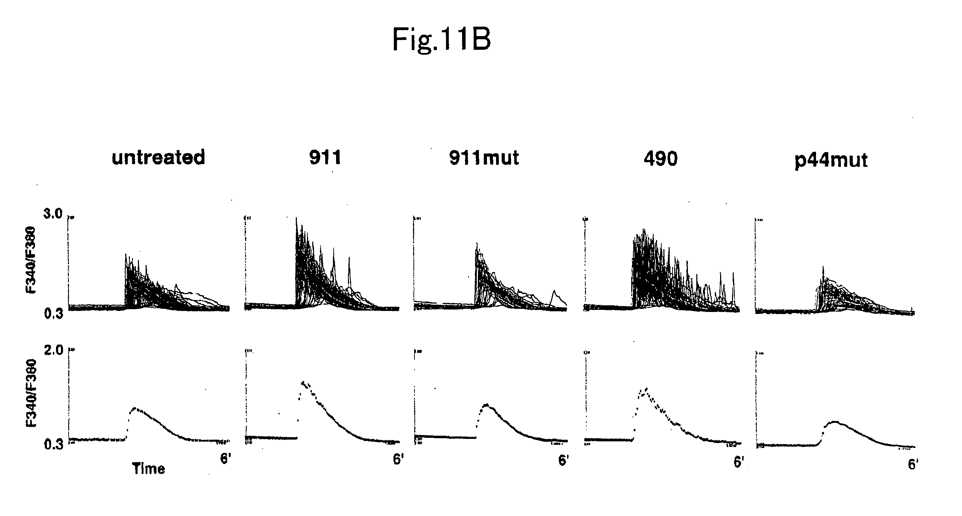

FIG. 11 shows that IP₃-induced calcium release of HeLa cells was increased when the expression of IRBIT was suppressed with RNA interference. (A) HeLa cells were transfected with 40 nM of small interference RNA (siRNA) against IRBIT (911 and 490) or control siRNA (911mut and p44mut) with LipofectAMINE2000 (Invitrogen). After two days, cells were harvested, and processed for western blotting with anti-IRBIT, anti-IP₃R1, anti-IP₃R2, anti-IP₃R3, anti-SERCA2, anti-calnexin, or anti-β-actin. (B) HeLa cells treated with siRNA as in (A) were loaded with 3 µM Fura-2-AM (Molecular Probes) for 20 min at 37 °C. Fluorescent images were obtained by alternate excitation at 340 and 380 nm. Cells were stimulated with 10 µM ATP.

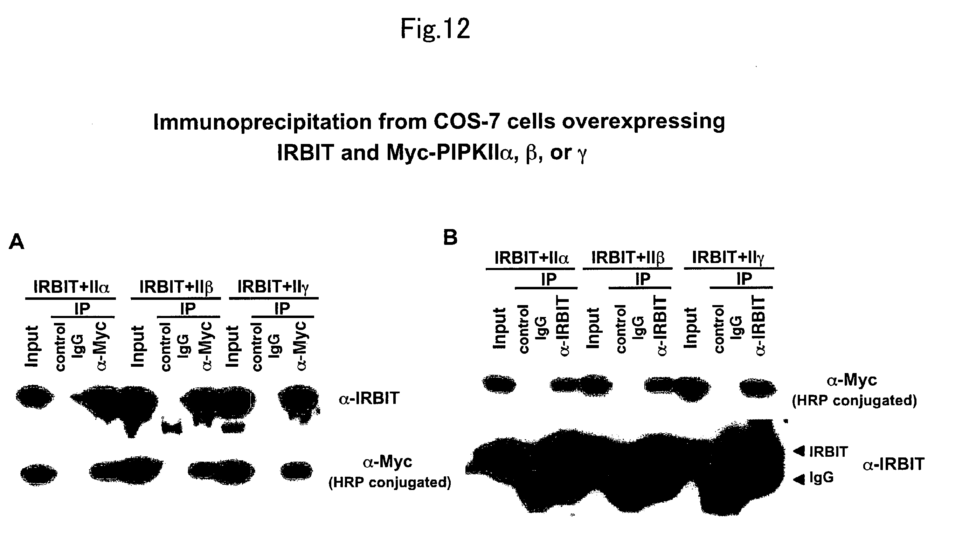

FIG. 12 shows that IRBIT interacted with type II phosphatidylinositol phosphate kinase. (A and B) IRBIT was transfected into Cos-7 cells with Myc-tagged type II phosphatidylinositol phosphate kinase α, β or γ (PIPKII α, β or γ). After two days, cells were lysed in lysis buffer, followed by centrifugation (100,000 x g, 30 min). The supernatants were incubated with 3 µg of anti-IRBIT antibody, rabbit IgG, mouse anti-Myc antibody, or mouse IgG for 1 h at 4 °C. After adding 5 µl of Protein G beads and another 1-h incubation, the beads were washed five times with lysis buffer and analyzed by Western blotting with anti-IRBIT antibody or HRP conjugated anti-Myc antibody. Immunoprecipitation of Myc-PIPKII α, β or γ with anti-Myc antibody co-precipitated IRBIT (A). In the reciprocal experiments, immunoprecipitation of IRBIT with anti-IRBIT antibody precipitated Myc-PIPKII α, β or γ .

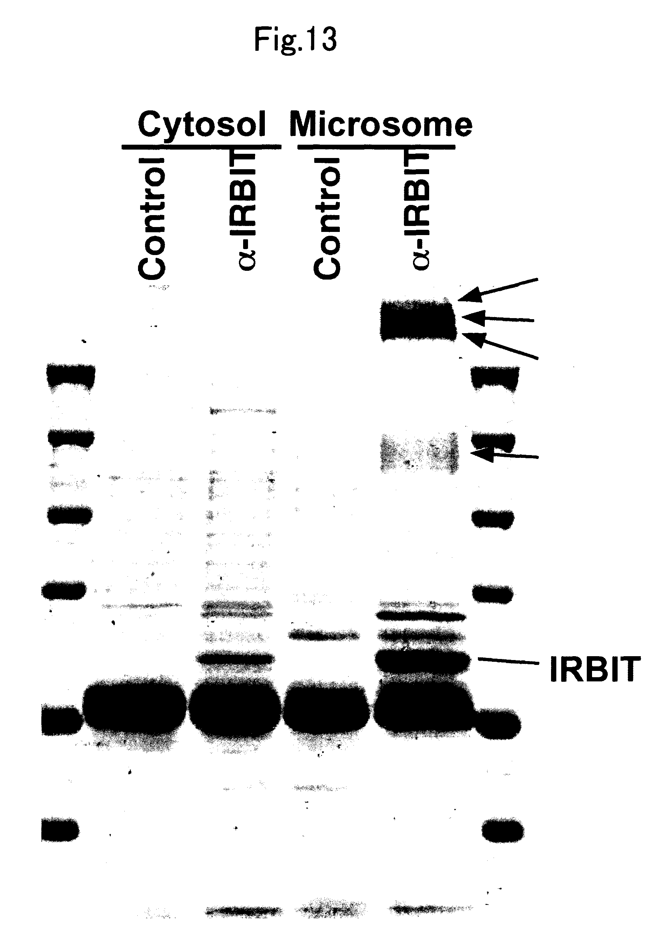

FIG. 13 shows that IRBIT interacted with sodium bicarbonate cotransporter. Mouse cerebellar cytosol fraction or detergent extract of microsome fraction was immunoprecipitated with 50 µg of anti-IRBIT antibody or rabbit IgG. Immunoprecipitates were separated by 10% SDS-PAGE gel, and stained with Coomassie Brilliant Blue. The bands were excised from the gel and digested with lysyl endopeptidase and analyzed with mass spectrometry. Arrows indicate the bands of sodium bicarbonate cotransporter.

### EXAMPLES

The present invention will be more particularly described with reference to the examples, but the present invention is not limited by these examples.

### Method

### Preparation of IP₃R1 affinity column

The cDNA encoding the N-terminal region (amino acids 1-225) of mouse IP₃R1 was inserted into glutathione S-transferase (GST) fusion vector pGEX-KG (40). The GST-IP₃R1 (1-225) fragment was subcloned into the baculovirus transfer vector pBlueBac4.5 (Invitrogen). The region located downstream from *Sma* I site of the GST-IP₃R1 (1-225) was replaced with the *Sma* I-*Eco*R I fragment of mouse IP₃R1 (corresponding to amino acids 79-2217) to construct a GST-IP₃R1 (1-2217) vector (termed GST-EL). A fragment encoding GST alone was subcloned into pBlueBac4.5 as a control. Sf9 cells were cultured in TNM-FH medium supplemented with 10% fetal bovine serum at 27°C. Recombinant baculoviruses carrying GST-EL or GST gene were generated with Bac-N-Blue™ Transfection Kit (Invitrogen) according to the instructions thereof. GST-EL and GST were expressed in 2×10⁸ Sf9 cells by infecting recombinant baculoviruses at a multiplicity of infection of 5, and incubating for 48 hours. The cells were harvested and stored at -80°C. The cryopreserved cells were suspended in 10 mM Hepes (pH 7.4), 100 mM NaCl, 2 mM EDTA, 1 mM 2-mercaptoethanol (2-ME), 0.1% Triton X-100, and protease inhibitors (1 mM phenylmethylsulfonyl fluoride (PMSF), 10 µM leupeptin, 2 µM pepstatin A and 10 µM E-64), and were homogenized with a glass-Teflon homogenizer (1000 rpm, 10 strokes). The homogenate was centrifuged at 20,000 × g for 30 minutes. The supernatant was incubated with 3 ml of glutathione-Sepharose 4B (Amersham Pharmacia Biotech) for 3 hours at 4°C. After washing 8 times with 40 ml of 10 mM Hepes (pH 7.4), 250 mM NaCl, 2 mM EDTA, 1 mM 2-ME, and 0.1% Triton X-100, GST-EL or GST coupled with glutathione-Sepharose was packed into columns and equilibrated with 10 mM Hepes (pH 7.4), 100 mM NaCl, 2 mM EDTA, 1 mM 2-ME, and 0.1% Triton X-100. Approximately 5 mg of GST-EL was immobilized.

### Purification and partial amino acid sequencing of IRBIT

Adult rat cerebella (approximately 5 g) were homogenized in 45 ml of a homogenize buffer (10 mM Hepes (pH 7.4), 20 mM Sucrose, 2 mM EDTA, 1 mM 2-ME and protease inhibitors) with a glass-Teflon homogenizer (950 rpm, 10 strokes). The homogenate was centrifuged at 1,000 × g for 10 minutes, and then the supernatant (S1 fraction) was further centrifuged at 100,000 × g for 60 minutes to obtain the cytosolic fraction (the supernatant) and the crude microsome (the pellet). The crude microsome was homogenized in 25 ml of a homogenize buffer containing 500 mM NaCl with a glass-Teflon homogenizer (1,200 rpm, 10 strokes), incubated on ice for 15 minutes, and then centrifuged at 100,000 × g for 60 minutes to obtain the high salt extract (supernatant) and the stripped-crude microsome (the pellet). The high salt extract was diluted 5-fold with a dilution buffer (10 mM Hepes (pH 7.4), 2 mM EDTA, 1 mM 2-ME, 0.01% Brij 35 and protease inhibitors). The diluted high salt extract was pre-cleared with glutathione-Sepharose and loaded into GST-EL affinity column equilibrated with a binding buffer (10 mM Hepes (pH 7.4), 100 mM NaCl, 2 mM EDTA and 1 mM 2-ME). A GST column was used as a control. The columns were washed with the binding buffer in a quantity 20 times greater than the volume of the column and proteins bound to the column were eluted with the binding buffer containing 50 µM IP₃ (Dojindo) and 0.05% Brij 35. The eluted material was concentrated, separated by 10% SDS-polyacrylamide gel electrophoresis (PAGE), and stained with Coomassie Brilliant Blue (CBB). The approximate 60-kDa protein band was excised from the gel and digested with lysyl endopeptidase (Wako) according to the known method (41). The digested peptides were separated using a C-18 reversed-phase column (µRPC C2/C18 SC 2.1/10, Amersham Pharmacia Biotech) connected to a SMART system (Amersham Pharmacia Biotech). The amino acid sequence of each peptide was determined by 494 precise protein sequencer (Applied Biosystems). Two peptide sequences, N-YSFMATVTK-C (SEQ ID NO: 3) and N-QIQFADDMQEFTK-C (SEQ ID NO: 4) were obtained.

### cDNA cloning of IRBIT

BLAST searches of the two peptide sequences above, obtained from the 60-kDa protein above against the non-redundant database, revealed that these sequences matched a sequence (Accession number: CAC09285) in the database. Based on the databases of mouse-expressed sequence tags (Accession numbers: AW229870 and BE282170), primers (5'-ATGTCGATGCCTGACGCGATGC-3' (SEQ ID NO: 5) and 5'-GCGTGGTTCATGTGGACTGGTC-3' (SEQ ID NO: 6)) homologous to the cDNA were synthesized. The cDNA of IRBIT was amplified by polymerase chain reaction (PCR) using mouse cerebellum oligo dT-primed, first-strand cDNA as a template. The PCR product was cloned in pBluescript II KS(+) (Stratagene) and sequenced. Sequences of the three independent clones were confirmed.

### Preparation of recombinant proteins

The cDNAs encoding the full-length and N-terminal region (amino acids 1 - 104) of IRBIT were subcloned into the *Escherichia coli* hexahistidine (His) fusion vector pET-23a(+) (Novagen) to generate IRBIT-His and IRBIT(1-104)-His expression vectors, respectively. The same cDNAs were subcloned into the GST fusion vector pGEX-4T-1 (Amersham Pharmacia Biotech) to generate GST-IRBIT and GST-IRBIT(1-104) expression vectors, respectively. The cDNA fragments encoding the amino acids 1-225, 1-343, 341-923, 600-1248, 916-1581 and 1553-1943 of the amino acid sequence (SEQ ID NO: 7) of mouse IP₃R1 were inserted into pGEX-KG to generate GST-Ia, GST-Iab, GST-IIab, GST-IIbIIIa, GST-IIIab and GST-IV expression vectors, respectively. The amino acids 1593-2217 of mouse IP₃R1 were inserted into pGEX-4T-1 to obtain GST-IV-Va expression vector. These fusion proteins were expressed in *Escherichia coli.* GST-EL was expressed in Sf9 cells as described above. The expressed His-tagged fusion proteins were purified using ProBond resin (Invitrogen), and GST fusion proteins were purified using glutathione-Sepharose. GST-IbIIa (amino acids 224-604 of mouse IP₃R1) and its site-directed mutants K508A and R441Q used herein were known previously (see Ref. 42; GST-IbIIa was referred to as G224 in the reference).

### Assays for enzyme activity

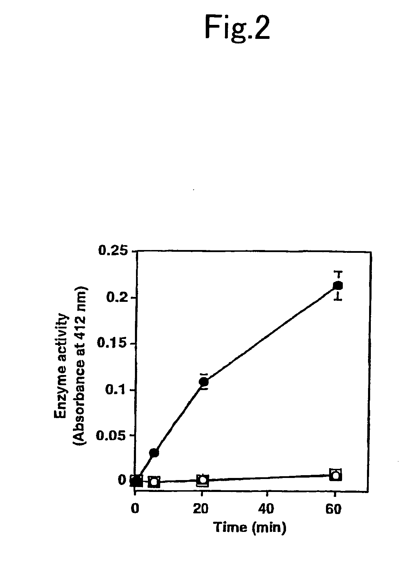

To assay S-adenosylhomocysteine hydrolase activity in the hydrolytic direction, the color developed by the reaction between the product (Homocysteine) and 5, 5'-dithiobis (2-nitrobenzoic acid) (Sigma) was measured spectroscopically using the purified IRBIT-His (3.0 µg), GST-IRBIT (4.3 µg), GST (1.3 µg) and rabbit S-adenosylhomocysteine hydrolase (Sigma) (2.4 µg), according to the known method (43). Absorbance at 412 nm was measured 0, 5, 20 and 60 minutes later using a DU 640 spectrophotometer (Beckman). Results are shown as the mean ± standard deviation from three independent experiments (Fig. 2).

### Production of affinity purified anti-IRBIT antibody

A Japanese White rabbit was immunized with the purified IRBIT(1-104)-His by subcutaneous injection with the complete Freund's adjuvant at 14-day intervals. The anti-IRBIT antisera were passed through a GST-IRBIT(1-104) covalently coupled with cyanogen bromide-activated Sepharose 4B (Amersham Pharmacia Biotech), and antibodies specifically bound to the column were eluted with 100 mM glycine-HCl (pH 2.5).

### Subcellular fractionation and immunoblotting

The cerebrum, cerebellum, heart, lung, liver, kidney, thymus, spleen, testis and ovary were removed from adult mice and S1 fraction was obtained as described above. The cytosolic fraction, crude microsome, high salt extract, and stripped-crude microsome of mouse cerebellum were obtained in a manner similar to the above method. Proteins with the amount indicated in Fig. 3 were subjected to 10% SDS-PAGE and transferred electrically onto polyvinylidene fluoride (PVDF) membrane. After blocking, the membranes were incubated with anti-IRBIT antibody (1 µg/ml) for 1 hour at room temperature, followed by blotting with horseradish peroxidase-conjugated donkey anti-rabbit IgG (Amersham Pharmacia Biotech). Immunoreactive bands were detected with an enhanced chemiluminescence detection system (Amersham Pharmacia Biotech).

### Generation and transfection of mammalian cell expression vectors

The cDNA encoding the full-length IRBIT was subcloned into the pcDNA3 (Invitrogen). The cDNA encoding the full-length IRBIT or its deletion mutants (amino acids 1-277, 1-104 and 105-530) were subcloned into the pEGFP-C1 (Clontech) to generate green fluorescent protein (GFP) fusion protein expression vectors. Mouse IP₃R1 expression vector pBact-STneoB-C1 used herein was known previously (44). Cos-7 cells were cultured in DMEM medium supplemented with 10% fetal bovine serum, penicillin, and streptomycin at 37°C. Transient transfections were performed using TransIT transfection reagents (Mirus) according to the instructions attached thereto. Two days after transfection, the transfected cells were used for immunoblotting, pulldown experiments, or immunostaining.

### In vitro binding experiments

Mouse cerebellar cytosolic fraction was diluted 2-fold with 10 mM Hepes (pH 7.4), 200 mM NaCl, 2 mM EDTA, 1 mM 2-ME and 0.02% Triton X-100. The high salt extract was diluted 5-fold with 10 mM Hepes (pH 7.4), 2 mM EDTA, 1 mM 2-ME and 0.01% Triton X-100. The diluted fractions (the final NaCl concentration of both fractions was 100 mM) were incubated with 20 µg of GST-EL or GST for 2 hours at 4°C. After adding 10 µl of glutathione-Sepharose and another 2 hours of incubation, the resins were washed 5 times with a wash buffer (10 mM Hepes (pH 7.4), 100 mM NaCl, 2 mM EDTA, 1 mM 2-ME, and 0.01% Triton X-100), and bound proteins were eluted with 20 mM glutathione. The eluted proteins were analyzed by Western blotting with anti-IRBIT antibody.

For dephosphorylation, the diluted high salt extract was incubated in the presence of or the absence of *Escherichia coli* alkaline phosphatase (Toyobo) with 2 mM MgCl₂ for 30 minutes at 37°C. 5 mM EDTA was added, and then the resultant was subjected to pulldown assay as described above.

For dissociation experiments, IRBIT in the diluted high salt extract was pulled down with GST-EL for precipitation and washed as described above. To resins, 100 µl of a wash buffer containing IP₃, inositol 4,5-bisphosphate (IP₂) (Dojindo), inositol 1,3,4,5-tetrakisphosphate (IP₄) (Calbiochem), inositol 1,2,3,4,5,6-hexakisphosphate (IP₆) (Calbiochem) or ATP (Amersham Pharmacia Biotech) (0.1, 0.3, 1, 3 or 10 µM) were added. After incubation on ice for 10 minutes, samples were centrifuged at 10,000 rpm for 1 minute, and the supernatant was subjected to immunoblot analysis with anti-IRBIT antibody or goat anti-GST antibody (Amersham Pharmacia Biotech). For quantification, Alexa 680-conjugated goat anti-rabbit IgG (Molecular Probes) was used as a secondary antibody. The fluorescence intensity of the immunoreactive bands of IRBIT was measured using the Odyssey infrared imaging system (Aloka). Quantitative data (the mean ± SD from at least three independent experiments) is expressed as a percentage of the amount of IRBIT in 10 µM IP₃ eluate.

For the determination of the IRBIT binding region and the critical amino acid residue of IP₃R1, the diluted high salt extract was subjected to pulldown assay as described above with 100 pmol of GST, GST-EL, GST-Ia, GST-Iab, GST-IbIIa, GST-IIab, GST-IIbIIIa, GST-IIIab, GST-IV, GST-IV-Va, K508A or R441 Q, and analyzed by Western blotting with anti-IRBIT antibody.

For the determination of the IP₃R1-interacting region of IRBIT, Cos-7 cells expressing GFP-tagged full-length IRBIT or its truncated mutants were lysed in a lysis buffer (10 mM Hepes (pH7.4), 100 mM NaCl, 2 mM EDTA, 1 mM 2-ME, 0.5% Nonidet P-40, and protease inhibitor) for 30 minutes at 4°C, followed by centrifugation (100,000 x g, 30 minutes). The supernatants were subjected to pulldown assay with GST-EL or GST as described above, and bound proteins were subjected to immunoblot analysis with anti-GFP antibody (Medical & Biological Laboratories).

### Indirect immunofluorescence and confocal microscopy

Transfected Cos-7 cells cultured on glass cover slips, washed once in phosphate-buffered saline (PBS), fixed in 4% formaldehyde-containing PBS for 15 minutes, permeabilized in 0.1% Triton X-100-containing PBS for 5 minutes, and then blocked in PBS containing 2% goat serum for 60 minutes at room temperature. For washing out cytosolic proteins, the cell membranes of transfected cells were punctured in an ice-cold permeabilization buffer (80 mM PIPES (pH 7.2), 1 mM MgCl₂, 1 mM EGTA and 4% polyethylene glycol) containing 0.1% saponin, and washed twice with the ice-cold permeabilization buffer, followed by fixation. The cells were allowed to react with rabbit anti-IRBIT antibody (1 µg/ml, room temperature, 60 minutes) and rat anti-IP₃R1 antibody 18A10 (45) (overnight at 4°C). After four times of 5 minutes of washing with PBS, Alexa 488-conjugated goat anti-rabbit IgG and Alexa 594-conjugated goat anti-rat IgG (Molecular Probes) were applied to reaction for 45 minutes at 37°C. After 4 instances of washing (5 minutes each) with PBS, the cover slips were mounted with Vectashield (Vector Laboratories) and observed via IX-70 confocal fluorescence microscopy (Olympus) with a 60x objective lens.

### Immunoprecipitation

Detergent-solubilized extract from the crude microsome was prepared with 1% Nonidet P-40-containing 50 mM Hepes (pH 7.4), 1 mM MgCl₂, and protease inhibitors for 30 minutes at 4°C, and centrifuged at 20,000 × g for 30 minutes. The supernatant was diluted 10-fold in an immunoprecipitation buffer (10 mM Hepes (pH 7.4), 150 mM NaCl, 1 mM MgCl₂, and 1% Nonidet P-40) and incubated with rabbit anti-IRBIT antibody (3 µg) or control rabbit IgG (3 µg) for 2 hours at 4°C. Protein G Sepharose 4 fast flow (Amersham Pharmacia Biotech) was added for reaction with immune complexes for 2 hours at 4°C. Resins were washed three times with an immunoprecipitation buffer and subjected to Western blotting with anti-IRBIT antibody or mouse anti-IP₃R1 antibody KM1112 (47).

### Results

### Purification and cDNA cloning of a novel IP₃R-binding protein

To identify IP₃ receptor-interacting molecules, the N-terminal region of 2217 amino acid residues encoding the greater part of the cytoplasmic portion of mouse IP₃R1 containing the IP₃ binding domain and regulatory domain (10, 11) was used as a fusion protein (GST-EL) with GST. GST-EL and GST were expressed by the baculovirus/Sf9 cell system and conjugated to glutathione-Sepharose. The fraction extracted with a high salt buffer (containing 500 mM NaCl) from the crude microsome of cerebella was thought to be enriched with membrane-bound proteins. The fraction was loaded into a glutathione-Sepharose affinity column in which GST-EL or GST was immobilized. To detect proteins which were dissociated from the IP₃ receptor in the presence of IP₃, the proteins bound to the affinity columns were eluted using 50 µM IP₃. A protein with a molecular mass of approximately 60 kDa was eluted from the GST-EL column (Fig. 1A), but not from the GST column (data not shown), suggesting that the protein specifically binds to IP₃R1. Two peptide sequences derived from the 60-kDa protein were determined. BLAST searches against non-redundant databases revealed that these two sequences matched the sequence of a human-derived cDNA in the database. Based on the sequence information of mouse-expressed sequence tags homologous to this cDNA, the cDNA of the above 60-kDa protein was obtained by reverse transcriptase-PCR from a mouse cerebellum. The predicted amino acid sequence of the cloned cDNA revealed a protein composed of 530 amino acid residues (Fig. 1B), with a calculated molecular mass of 58.9 kDa, which was almost the same as its predicted molecular mass estimated by SDS-PAGE. The 60-kDa molecule is termed IRBIT.

The C-terminal region (amino acids 105-530) of IRBIT was shown to be homologous (51% identical and 74% similar) to the methylation pathway enzyme S-adenosylhomocysteine hydrolase (EC3.3.1.1.) (48) (Fig. 1, C and D). The region on the N-terminal side (amino acid residues 1 to 104) of IRBIT had no homology with known proteins and contained a serine-rich region (amino acid residues 62 to 103) (Fig. 1B and D). Motif searches of the IRBIT sequence revealed the presence of a putative coiled-coil motif (amino acids 111-138) and a putative NAD+ binding region (amino acids 314-344) (Fig. 1B and D). There were 17 potential phosphorylation sites for protein kinases such as casein kinase II, PKC, PKA/PKG and tyrosine kinases, out of which seven sites were concentrated on the N-terminal side (Fig. 1B). Putative membrane-spanning regions and signal sequences were not found.

Because IRBIT had homology with S-adenosylhomocysteine hydrolase, which catalyzes the reversible hydrolysis of S-adenosylhomocysteine to adenosine and homocysteine, whether IRBIT had the same enzyme activity was investigated using recombinant IRBIT expressed in *Escherichia coli.* C-terminally His-tagged IRBIT (IRBIT-His) and N-terminally GST-tagged IRBIT (GST-IRBIT) were purified, and their enzyme activities were measured in hydrolysis direction. As shown in Fig. 2, neither of the recombinant IRBITs had enzyme activity. Thus it can be concluded that IRBIT has no S-adenosylhomocysteine hydrolase activity. Crystallographic studies and site-directed mutagenesis studies (43, 52-56) have determined the amino acid residues of S-adenosylhomocysteine hydrolase involved in substrate binding or NAD+ binding. The fact that IRBIT showed no S-adenosylhomocysteine hydrolase activity is probably due to substitution of amino acids important for substrate binding of S-adenosylhomocysteine hydrolase, such as Lysine-54, Phenylalanine-302 and Histidine-353 (Fig. 1C). However, the possibility that IRBIT has enzyme activity with different substrate specificity is not excluded.

### Tissue distribution and subcellular localization of IRBIT

An affinity-purified antibody against the N-terminal region of IRBIT (Fig. 1B, boxed) was generated. To examine the specificity of this antibody, the cDNA of IRBIT was transfected into Cos-7 cells and the cell lysates were analyzed by immunoblotting with the anti-IRBIT antibody. As shown in Fig. 3A, the anti-IRBIT antibody recognized only a protein located approximately at 60 kDa. The molecular mass of the exogeneously expressed IRBIT (Fig. 3A, lane 1) was the same as that of the endogenous protein in Cos-7 (Fig. 3A, lane 3), confirming that the above cDNA encodes the full-length IRBIT. The expression of IRBIT in mouse tissue was examined by immunoblot analysis with this anti-IRBIT antibody. IRBIT was detected ubiquitously, with the highest expression in the cerebrum and cerebellum (Fig. 3B).

Next, subcellular localization of IRBIT was examined by fractionation of mouse cerebellum. IRBIT was present in both cytosolic and crude microsome fractions (Fig. 3C, lanes 2 and 3). The crude microsome was further separated in a peripherally membrane-bound fraction (the fraction from which IRBIT had been originally purified) and a stripped-membrane fraction. As shown in Fig. 3C, IRBIT in the crude microsome was partially extracted with a high salt buffer (Fig. 3C, lane 4). In contrast, IP₃R1, which is an integral membrane protein of the endoplasmic reticulum, was not extracted at all (Fig. 3C, lower panel). These results indicate that IRBIT is present in cytoplasm and also binds to a membrane.

### IRBIT in high salt extract interacted with IP₃R1 and the N-terminal region of IRBIT was essential for the interaction.

IRBIT was present in both cytosolic and peripherally membrane-bound fractions of mouse cerebellum (Fig. 3C). Whether IRBIT in these fractions interacted with IP₃R1 in vitro was examined by the GST pulldown method. The cytosol and high salt extract (specifically, the membrane-bound fraction) from the crude microsome of mouse cerebella were incubated with GST-EL or GST and binding of IRBIT to the recombinant proteins was analyzed by immunoblotting with anti-IRBIT antibody. As shown in Fig. 4A, IRBIT in the high salt extract interacted with GST-EL, but not with GST (Fig. 4A, lanes 5 and 6). In contrast, IRBIT in the cytosolic fraction did not interact with GST-EL (Fig. 4A, lane 3). The fact that these results were neither due to the buffer nor to other small molecules contained in the fraction was confirmed by conducting similar experiments after buffer exchange by dialysis. It was speculated that the binding with the IP₃ receptor is regulated by the post-translational modification of IRBIT such as phosphorylation.

To examine the effect of phosphorylation, the high salt extract was treated with alkaline phosphatase, a nonspecific phosphatase, and then incubated with GST-EL or GST. As shown in Fig. 4B, IRBIT in the high salt extract did not interact with GST-EL after phosphatase treatment (Fig. 4B, lane 6). This result suggests the possibility that the phosphorylation of IRBIT may be necessary for IRBIT to associate with IP₃R1. However, at this point in time the possibility that phosphorylation of other proteins may regulate the interaction of these proteins is not excluded. Based on the fact that the interaction was not observed after the alkaline phosphatase treatment of IRBIT, it is assumed that the interaction between IRBIT and the IP₃ receptor is also regulated by phosphorylation. Next, to specify the region of IRBIT required for interaction with IP₃R1, the GST pulldown method was performed using GFP-tagged deletion mutants of IRBIT. As shown in Fig. 5B, both GFP-IRBIT and GFP-IRBIT (1 to 277) bound to GST-EL efficiently (Fig. 5B, lanes 3 and 6). Although GFP-IRBIT (1-104) interacted with GST-EL, the interaction was much weaker than that between GFP-IRBIT and GFP-IRBIT (1-277) (Fig. 5B, comparison of lanes 7 and 9 with lanes 1 and 3, and with lanes 4 and 6). In contrast, GFP-IRBIT (105-530), which lacked the N-terminal region, did not interact with GST-EL (Fig. 5B, lanes 12 and 15). These results demonstrate that the N-terminal region of IRBIT was essential for the interaction with IP₃R1, and approximately 170 amino acid residues (105-277) having a coiled-coil structure were important for stabilizing the interaction.

Based on the fact that IRBIT has 17 potential phosphorylation sites, seven of which are concentrated on the above N-terminal region, which is necessary for the interaction with the IP₃ receptor, it is predicted that phosphorylation may be involved in the interaction with the IP₃ receptor. It was hypothesized based on these findings that the dephosphorylated form of IRBIT is free in cytosol, whereas the phosphorylated form of IRBIT is membrane-bound via the interaction with the IP₃ receptor. Although it could not be demonstrated yet, the interaction between IRBIT and the IP₃ receptor may be dualistically regulated by IP₃, and by either direct or indirect phosphorylation.

### IRBIT co-localized with IP₃R1 on endoplasmic reticulum in Cos-7 cells

To investigate the subcellular localization of IRBIT, Cos-7 cells over-expressing IRBIT and IP₃ receptor were analyzed by confocal immunofluorescence microscopy. IRBIT was diffusely distributed in cytoplasm, with no immunoreactivity in nucleus (Fig. 6A). Because IRBIT was shown to be present in both cytosolic and crude microsome fractions by biochemical fractionation experiments (Fig. 3C), visualization and observation of only the membrane-bound population of IRBIT were attempted. For this purpose, the cell membranes of Cos-7 cells co-expressing IRBIT and the IP₃ receptor were permeabilized using saponin to wash out cytosolic IRBIT prior to fixation. As shown in Fig. 6B, IRBIT was distributed in a reticular pattern (Fig. 6B, left panels), indicating that membrane-bound IRBIT was distributed on the endoplasmic reticulum. The immunoreactivity of IRBIT intensively overlapped that of IP₃R1 (Fig. 6B, middle panels and right panels). The staining pattern of IP₃R1 was not altered by permeabilization with saponin (data not shown). When IP₃R1 and GFP-IRBIT were co-expressed, and the fluorescence of GFP was observed, the same results were observed (Fig. 6C and D). These results indicate that IRBIT interacted with IP₃R1 on the endoplasmic reticulum in the over-expressing Cos-7 cells. When GFP-IRBIT (105-530), which did not interact with GST-EL because of the lack of the N-terminal region, and IP₃R1 were co-expressed in Cos-7 cells to confirm that the localization was specific, GFP-IRBIT (105-530) was distributed in the cytosol and nucleus (Fig. 6E), unlike GFP-IRBIT. IRBIT does not have any sequence predicted to constitute nuclear localization signals, so that the mechanism of nuclear localization is unclear. When IRBIT located in cytosol was washed out by saponin treatment, GFP-IRBIT (105-530) localized only in the nucleus and the co-localization of the GFP-IRBIT with IP₃R1 was not confirmed (Fig. 6F). This result was consistent with the results obtained by biochemical techniques to the effect that the N-terminal region of IRBIT was essential for binding to IP₃R1 (Fig. 5B).

### IRBIT interacted with IP₃R1 in vivo

To confirm an association between IRBIT and IP₃R1 in tissue, co-immunoprecipitation was performed using a mouse cerebellum. Detergent extracts of the crude microsome of the mouse cerebellum were immunoprecipitated with anti-IRBIT antibody and the immunoprecipitates were analyzed by immunoblotting with anti-IP₃R1 antibody. IP₃R1 was co-immunoprecipitated with anti-IRBIT antibody, but not with a control antibody (Fig. 7). These results indicate the in vivo association of IRBIT with IP₃R1 in the cerebellum.

### Physiological concentration of IP₃ selectively dissociated IRBIT from IP₃R1

IRBIT was originally identified in the eluate from the GST-EL column with 50 µM IP₃ (Fig. 1A), suggesting that IP₃ disrupted the interaction between IRBIT and IP₃R1. However, the intracellular concentration of IP₃ was at the level of several micrometers, even when it was elevated by stimulation (50). The IP₃ concentration of 50 µM used in the above elution is higher than the physiological range. Thus, whether or not the interaction between the proteins was disrupted by IP₃ at a physiological concentration was examined.

IRBIT in the high salt extract from the mouse cerebellar crude microsome was trapped with GST-EL. Then elution was attempted with 0.1 to 10 µM IP₃, and other inositolpolyphosphates, IP₂, IP₄, IP₆ or ATP. As shown in Fig. 8A, IP₃ dissociated IRBIT from GST-EL in a concentration-dependent manner (Fig. 8Aa, lower panel). It was confirmed that GST-EL was not detected (Fig. 8Aa, upper panel), even with longer exposure. The EC50 (IP₃ concentration required for half (50%)-maximal dissociation of IRBIT from GST-EL) was approximately 0.5 µM, which was within the physiological range of IP₃ concentration (50) (Fig. 8B). The dissociation efficiency of IP₃ was approximately 50 times greater than those of other inositol polyphosphates. ATP did not dissociate IRBIT even at a concentration of 10 µM. These results indicate that IRBIT was dissociated from IP₃R1 selectively with physiological concentrations of IP₃.

### IRBIT interacted with the IP₃-binding region of IP₃R1 and Lys-508 of IP₃R1 was essential for the interaction with both IRBIT and IP₃

To examine which region of the IP₃-binding region or the regulatory region of IP₃R1 was essential for the interaction with IRBIT, 8 types of IP₃R1 deletion mutants constructed as GST fusion proteins based on the domain structure of IP₃R1 (51) were used (Fig. 9A). As shown in Fig. 9B, GST-IbIIa (amino acids 226-604) containing the IP₃ binding core region (amino acids 226-576) (11) bound to IRBIT to the same extent as GST-EL. In contrast, other GST fusion proteins, including GST-Iab and GST-IIab, did not interact with IRBIT. Next, site-directed mutagenesis analysis was performed to determine the important amino acid residues of IP₃R1 for the interaction with IRBIT. Lysine at position 508 of IP₃R1 (Lys-508) is known to be an essential amino acid residue for IP₃ binding (12), and substitution of the lysine residue of GST-IbIIa with alanine (K508A) is known to show a significant loss in IP₃ binding affinity (42). Conversely, R441Q, in which arginine residue at position 441 of GST-IbIIa was substituted with glutamine, is known to have higher binding affinity for IP₃ than that of GST-IbIIa (42). When the pulldown assay was performed using these recombinant proteins, IRBIT bound to GST-IbIIa and R441Q to the same extent, but did not bind to K508A (Fig. 9C). These results indicate that lysine at position 508 of IP₃R1 is necessary not only for the interaction with IP₃, but also for the interaction with IRBIT, supporting the results that IP₃ disrupted the interaction between IRBIT and IP₃R1.

It is concluded based on the above results that IRBIT is normally associated with IP₃R1, and is dissociated from IP₃R1 when IP₃ concentration is elevated by extracellular stimulation. IRBIT is shown to be a sole IP₃ receptor-binding protein, whose interaction with IP₃ receptor can be regulated by IP₃.

### IRBIT lowered the affinity of IP₃R to IP₃ in a phosphorylation dependent manner

The effect of IRBIT on binding of IP₃ to IP₃R was examined. GST-EL (0.2 µg) was incubated with 0.1, 1, or 10 µg of purified His-tagged IRBIT expressed in Sf9 cells or *E. coli* in a solution containing 50 mM Tris-HCl (pH 8.0), 1 mM EDTA, and 1 mM 2-mercaptoethanol, for 30 min on ice. Then 8.7 nM [³H]IP₃ (PerkinElmer Life Sciences) was added to the samples and incubated for 10 min on ice (total volume was 50 µl). The samples were mixed with 2 µl of 50 mg/ml γ-globulin and 50 µl of 30 % PEG6000, 50 mM Tris-HCl (pH 8.0), and incubated for 5 min on ice. Non-specific binding was measured in the presence of 10 µM cold IP₃. After centrifugation at 20,000 x g for 5 min, the precipitate was dissolved in SOLVABLE™ (Packard) and the radioactivity was measured with a liquid scintillation counter (Beckman Coulter). The result is shown in Fig 10 (A). His-IRBIT expressed in Sf9 dose-dependently suppressed the IP₃ binding of GST-EL. Furthermore, the dephosphorylation of IRBIT by alkaline phosphatase treatment reduced effect of IRBIT on IP₃ binding of GST-EL (Fig 10 (A)). Together with the result of (A), this result suggests that phosphorylation of IRBIT is necessary to suppress the IP₃ binding of IP₃R. Fig 10 C and D shows the plot of Scatchard analysis. 0.1 µg of GST-EL was incubated with or without 1 µg of purified His-tagged IRBIT expressed in Sf9 cells as described in (A). Then 2.17 nM [³H]IP₃ and 2-500 nM cold IP₃ were added to the samples and incubated for 10 min on ice (total volume was 100 µl). Non-specific binding was measured in the presence of 10 µM cold IP₃. [³H]IP₃ binding to GST-EL was measured as described in (A). His-IRBIT decreased the affinity of GST-EL to IP₃. Bmax was not changed.

### IP₃-induced calcium release of HeLa cells was increased when the expression of IRBIT was suppressed with RNA interference

RNA interference experiment showed that IP₃-induced calcium release of HeLa cells was increased when the expression of IRBIT was suppressed with RNA interference (Fig 11). HeLa cells were transfected with 40 nM of small interference RNA (siRNA) against IRBIT (911 and 490) or control siRNA (911mut and p44mut) with LipofectAMINE2000 (Invitrogen). After two days, cells were harvested, and processed for western blotting with anti-IRBIT, anti-IP₃R1, anti-IP₃R2, anti-IP₃R3, anti-SERCA2, anti-calnexin, or anti-β-actin. As seen Fig 11A, expression of IRBIT was specifically suppressed by RNA interference with siRNA 911 and 490. In addtion, HeLa cells treated with siRNA as in (A) were loaded with 3 µM Fura-2-AM (Molecular Probes) for 20 min at 37 °C. Fluorescent images were obtained by alternate excitation at 340 and 380 nm. Cells were stimulated with 10 µM ATP. Suppression of expression of IRBIT with siRNA 911 or 490 increased the calcium release with agonist stimulation.

### IRBIT interacted with type II phosphatidylinositol phosphate kinase.

IRBIT was transfected into Cos-7 cells with Myc-tagged type II phosphatidylinositol phosphate kinase α, β or γ (PIPKII α, β or γ). After two days, cells were lysed in lysis buffer, followed by centrifugation (100,000 x g, 30 min). The supernatants were incubated with 3 µg of anti-IRBIT antibody, rabbit IgG, mouse anti-Myc antibody, or mouse IgG for 1 h at 4 °C. After adding 5 µl of Protein G beads and another 1-h incubation, the beads were washed five times with lysis buffer and analyzed by Western blotting with anti-IRBIT antibody or HRP conjugated anti-Myc antibody.

Immunoprecipitation of Myc-PIPKII α, β or γ with anti-Myc antibody co-precipitated IRBIT (Fig. 12 A). In the reciprocal experiments, immunoprecipitation of IRBIT with anti-IRBIT antibody precipitated Myc-PIPKII α, β or γ (Fig. 12 B). These results indicated that IRBIT interacted with all three isoforms of PIPKII in overexpressing cells.

### IRBIT interacted with sodium bicarbonate cotransporter.

Mouse cerebellar cytosol fraction or detergent extract of microsome fraction was immunoprecipitated with 50 µg of anti-IRBIT antibody or rabbit IgG. Immunoprecipitates were separated by 10% SDS-PAGE gel, and stained with Coomassie Brilliant Blue (Fig. 13). The bands were excised from the gel and digested with lysyl endopeptidase and analyzed with mass spectrometry. Arrows indicate the bands of sodium bicarbonate cotransporter.

In summery, the IRBIT protein that we have found herein interacts with the IP₃ receptor and the interaction is disrupted by IP₃ in an IP₃ concentration-dependent manner. Thus, the IRBIT was shown to be a useful protein molecule as an indicator for detecting and quantifying IP₃ both intracellularly and in a cell-free system. Further, since the sequence from amino acid residues 1 to 104 of the amino acid sequence of IRBIT is a region essential for binding to IP₃, it is considered that in addition to IRBIT, a protein containing at least the amino acid sequence (1-104) is also useful as an indicator for IP₃.

All publications, patents and patent applications cited herein are incorporated herein by reference in their entirety.

### References

1. Berridge, M. J. (1993) Nature 361, 315-325
2. Berridge, M. J., Lipp, P., and Bootman, M. D. (2000) Nat. Rev. Mol. Cell Biol. 1, 11-21
3. Furuichi, T., and Mikoshiba, K. (1995) J. Neurochem. 64, 953-960
4. Patel, S., Joseph, S. K., and Thomas, A. P. (1999) Cell Calcium 25, 247-264
5. Furuichi, T., Yoshikawa, S., Miyawaki, A., Wada, K., Maeda, N., and Mikoshiba, K. (1989) Nature 342, 32-38
6. Südhof, T. C., Newton, C. L., Archer, B. T., III, Ushkaryov, U. A., and Mignery, G. A. (1991) EMBO J. 10, 3199-3206
7. Blondel, O., Takeda, J., Janssen, H., Seino, S., and Bell, G. I. (1993) J. Biol. Chem. 268, 11356-11363
8. Worley, P. F., Baraban, J. M., Colvin, J. S., and Snyder, S. H. (1987) Nature 325, 159-161
9. Furuichi, T., Simon-Chazottes, D., Fujino, I., Yamada, N., Hasegawa, M., Miyawaki, A., Yoshikawa, S., Guénet, J.-L., and Mikoshiba, K. (1993) Recept. Channels 1, 11-24
10. Mignery, G. A., and Südhof, T. C. (1990) EMBO J. 9, 3893-3898
11. Miyawaki, A., Furuichi, T., Ryou, Y., Yoshikawa, S., Nakagawa, T., Saitoh, T., and Mikoshiba, K. (1991) Proc. Natl. Acad. Sci. USA 88, 4911-4915
12. Yoshikawa, F., Morita, M., Monkawa, T., Michikawa, T., Furuichi, T., and Mikoshiba, K. (1996) J. Biol. Chem. 271, 18277-18284
13. Zhu, C.-C., Furuichi, T., Mikoshiba, K., and Wojcikiewicz, R. J. H. (1999) J. Biol. Chem. 274, 3476-3484
14. Zhu, C.-C., and Wojcikiewicz, R. J. H. (2000) Biochem. J. 348, 551-556
15. Berridge, M. J., Bootman, M. D., and Lipp, P. (1998) Nature 395, 645-648
16. Thrower, E. C., Hagar, R. E., and Ehrlich, B. E. (2001) Trends Pharmacol. Sci. 22, 580-586
17. Mackrill, J. J. (1999) Biochem. J. 337, 345-361
18. Patel, S., Morris, S. A., Adkins, C. E., O'beirne, G., and Taylor, C. W. (1997) Proc. Natl. Acad. Sci. USA 94, 11627-11632
19. Michikawa, T., Hirota, J., Kawano, S., Hiraoka, M., Yamada, M., Furuichi, T., and Mikoshiba, K. (1999) Neuron 23, 799-808
20. Cameron, A. M., Steiner, J. P., Sabatini, D. M., Kaplin, A. I., Walensky, L. D., and Snyder, S. H. (1995) Proc. Natl. Acad. Sci. USA 92, 1784-1788
21. Cameron, A. M., Nucifora, F. C., Jr, Fung, E. T., Livingston, D. J., Aldape, R. A., Ross, C. A., and Snyder, S. H. (1997) J. Biol. Chem. 272, 27582-27588
22. Dargan, S. L., Lea, E. J. A., and Dawson, A. P. (2002) Biochem. J. 361, 401-407
23. Bultynck, G., De Smet, P., Weidema, A. F., Ver Heyen, M., Maes, K., Callewaert, G., Missiaen, L., Parys, J. B., and De Smedt, H. (2000) J. Physiol. 525, 681-693
24. Bultynck, G., De Smet, P., Rossi, D., Callewaert, G., Missiaen, L., Sorrentino, V., De Smedt, H., and Parys, J. B. (2001) Biochem. J. 354, 413-422
25. Cameron, A. M., Steiner, J. P., Roskams, A. J., Ali, S. M., Ronnett, G. V., and Snyder, S. H. (1995) Cell 83, 463-472
26. Joseph, S. K., and Samanta, S. (1993) J. Biol. Chem. 268, 6477-6486
27. Bourguignon, L. Y. W., Jin, H., Iida, N., Brandt, N. R., and Zhang, S. H. (1993) J. Biol. Chem. 268, 7290-7297
28. Hayashi, T., and Su, T.-P. (2001) Proc. Natl. Acad. Sci. USA 98, 491-496
29. Yoo, S. H., So, S. H., Kweon, H. S., Lee, J. S., Kang, M. K., and Jeon, C. J. (2000) J. Biol. Chem. 275, 12553-12559
30. Yoo, S. H., and Jeon, C. J. (2000) J. Biol. Chem. 275, 15067-15073
31. Thrower, E. C., Park, H. Y., So, S. H., Yoo, S. H., and Ehrlich, B. E. (2002) J. Biol. Chem. 277, 15801-15806
32. Schlossmann, J., Ammendola, A., Ashman, K., Zong, X., Huber, A., Neubauer, G., Wang, G.-X., Allescher, H.-D., Korth, M., Wilm, M., Hofmann, F., and Ruth, P. (2000) Nature 404, 197-201
33. Jayaraman, T., Ondrias, K., Ondiasova, E., and Marks, A. R. (1996) Science 272, 1492-1494
34. Yokoyama, K., Su, I., Tezuka, T., Yasuda, T., Mikoshiba, K., Tarakhovsky, A., and Yamamoto, T. (2002) EMBO J. 21, 83-92
35. Yang, J., McBride, S., Mak, D.-O. D., Vardi, N., Palczewski, K., Haeseleer, F., and Foskett, J. K. (2002) Proc. Natl. Acad. Sci. USA 99, 7711-7716
36. Tu, J. C., Xiao, B., Yuan, J. P., Lanahan, A. A., Leoffert, K., Li, M., Linden, D. J., and Worley, P. F. (1998) Neuron 21, 717-726
37. Delmas, P., Wanaverbecq, N., Abogadie, F. C., Mistry, M., and Brown, D. A. (2002) Neuron 14, 209-220
38. Kiselyov, K., Mignery, G. A., Zhu, M. X., and Muallem, S. (1999) Mol. Cell 4, 423-429
39. Boulay, G., Brown, D. M., Qin, N., Jiang, M., Dietrich, A., Zhu, M. X., Chen, Z., Birnbaumer, M., Mikoshiba, K., and Birnbaumer, L. (1999) Proc. Natl. Acad. Sci. USA 96, 14955-14960
40. Guan, K., and Dixon, J. E. (1991) Anal. Biochem. 192, 262-267
41. Rosenfeld, J., Capdevielle, J., Guillemot, J. C., and Ferrara, P. (1992) Anal. Biochem. 203, 173-179
42. Uchiyama, T., Yoshikawa, F., Hishida, A., Furuichi, T., and Mikoshiba, K. (2002) J. Biol. Chem. 277, 8106-8113
43. Yuan, C.-S., Ault-Riché, D. B., and Borchardt, R. T. (1996) J. Biol. Chem. 271, 28009-28016
44. Miyawaki, A., Furuichi, T., Maeda. N., and Mikoshiba, K. (1990) Neuron 5, 11-18
45. Maeda, N., Niinobe, M., Nakahira, K., and Mikoshiba, K. (1988) J. Neurochem. 51, 1724-1730
46. Maeda, N., Niinobe, M., Inoue, Y., and Mikoshiba, K. (1989) Dev. Biol. 133, 67-76
47. Sugiyama, T., Furuya, A., Monkawa, T., Yamamoto-Hino, M., Satoh, S., Ohmori, K., Miyawaki, A., Hanai, N., Mikoshiba, K., and Hasegawa, M. (1994) FEBS Lett. 354, 149-154
48. Ogawa, H., Gomi, T., Mueckler, M. M., Fujioka, M., Backlund, P. S., Jr., Aksamit, R. R., Unson, C. G., and Cantoni, G. L. (1987) Proc. Natl. Acad. Sci. USA 84, 719-723
49. Dekker, J. W., Budhia, S., Angel, N. Z., Cooper, B. J., Clark, G. J., Hart, D. N. J., and Kato, M. (2002) Immunogenetics 53, 993-1001
50. Luzzi, V., Sims, C. E., Soughayer, J. S., and Allbritton, N. L. (1998) J. Biol. Chem. 273, 28657-28662
51. Yoshikawa, F., Iwasaki, H., Michikawa, T., Furuichi, T., and Mikoshiba, K. (1999) J. Biol. Chem. 274, 316-327
52. Turner, M. A., Yuan, C.-S., Borchardt, R. T., Hershfield, M. S., Smith, G. D., and Howell, P. L. (1998) Nat. struct. Biol. 5, 369-376
53. Hu, Y., Komoto, J., Huang, Y., Gomi, T., Ogawa, H., Takata, Y., Fujioka, M., and Takusagawa, F. (1999) Biochemistry 38, 8323-8333
54. Gomi. T., Takata, Y., Date, T., Fujioka, M., Aksamit, R. R., Backlund, P. S., Jr., and Cantoni, G. L. (1990) J. Biol. Chem. 265, 16102-16107
55. Aksamit, R. R., Backlund, P. S., Jr., Moos, M., Jr., Caryk, T., Gomi, T., Ogawa, H., Fujioka, M., and Cantoni, G. L. (1994) J. Biol. Chem. 269, 4084-4091
56. Ault-Riché, D. B., Yuan, C.-S., and Borchardt, R. T. (1994) J. Biol. Chem. 269, 31472-31478
57. Ichtchenko, K., Hata, Y., Nguyen, T., Ullrich, B., Missler, M., Moomaw, C., and Südhof, T. C. (1995) Cell 81, 435-443
58. Peles, E., Nativ, M., Campbell, P. L., Sakurai, T., Martinez, R., Lev, S., Clary, D. O., Schilling, J., Barnea, G., Plowman, G. D., Grumet, M., and Schlessinger, J. (1995) Cell 82, 251-260
59. Peifer, M., Berg, S., and Reynolds, A. B. (1994) Cell 76, 789-791
60. Supattapone, S., Worley, P. F., Baraban, J. M., and Snyder, S. H. (1988) J. Biol. Chem. 263, 1530-1534
61. Maeda, N., Niinobe, M., and Mikoshiba, K. (1990) EMBO J. 9, 61-67
62. Worley, P. F., Baraban, J. M., Supattapone, S., Wilson, V. S., and Snyder, S. H. (1987) J. Biol. Chem. 262, 12132-12136
63. Hannaert-Merah, Z., Coquil, J. F., Combettes, L., Claret, M., Mauger, J. P., and Champeil, P. (1994) J. Biol. Chem. 269, 29642-29649
64. Natsume, T., Hirota, J., Yoshikawa, F., Furuichi, T., and Mikoshiba, K. (1999) Biochem. Biophys. Res. Com. 260, 527-533
65. Yoshikawa, F., Uchiyama, T., Iwasaki, H., Tomomori-Satoh, C., Tanaka, T., Furuichi, T., and Mikoshiba, K. (1999) Biochem. Biophys. Res. Com. 257, 792-797
66. Wojcikiewicz, R. J. H., Furuichi, T., Nakade, S., Mikoshiba, K., and Nahorski, S. R. (1994) J. Biol. Chem. 269, 7963-7969
67. Wojcikiewicz, R. J. H. (1995) J. Biol. Chem. 270, 11678-11683
68. Bokkala, S., and Joseph, S. K. (1997) J. Biol. Chem. 272, 12454-12461
69. Oberdorf, J., Webster, J. M., Zhu, C. C., Luo, S. G., and Wojcikiewicz, R. J. H. (1999) Biochem. J. 339, 453-461
70. Pollok, B. A., and Heim, R. (1999) Trends Cell Biol. 9, 57-60
71. Adams, S. R., Harootunian, A. T., Buechler, Y. J., Taylor, S. S., and Tsien, R. Y. (1991) Nature 349, 694-697
72. Miyawaki, A., Llopis, J., Heim, R., McCaffery, J. M., Adams, J. A., Ikura, M., and Tsien, R. Y. (1997) Nature 388, 882-887
73. Hirose, K., Kadowaki, S., Tanabe, M., Takeshima, H., and Iino, M. (1999) Science 284, 1527-1530

## Claims

1. A protein (a), (b) or (c) as shown below:
(a) a protein (IRBIT), comprising an amino acid sequence represented by SEQ ID NO: 1,
(b) a protein, comprising an amino acid sequence of the 1-104 of SEQ ID NO: 1, and
(c) a protein, comprising an amino acid sequence derived from the amino acid sequence of SEQ ID NO: 1 or an amino acid sequence of the 1-104 of SEQ ID NO: 1, which contains a deletion, substitution or addition of at least one of amino acid residues, and binding to an IP₃ receptor.

2. A gene, encoding the protein of claim 1.

3. A gene, comprising a DNA (a), (b) or (c) as shown below:
(a) a DNA, comprising a nucleotide sequence of SEQ ID NO: 2,
(b) a DNA, comprising a nucleotide sequence of the 1 to 312 of SEQ ID NO: 2, and
(c) a DNA, hybridizing under stringent conditions to a DNA that is complementary to the DNA comprising the nucleotide sequence of (a) or (b), and encoding a protein that binds to the IP₃ receptor.

4. A vector, containing the gene of claim 2 or 3.

5. The vector of claim 4, wherein a promoter is operably linked to the gene of claim 2 or 3.

6. The vector of claim 5, wherein the gene of claim 2 or 3 is linked in-frame with a gene encoding a fluorescent protein or a photoprotein.

7. The vector of claim 6, wherein the fluourescent protein is selected from the group consisting of a green fluorescent protein (GFP), a cyan fluorescent protein (CFP), yellow fluorescent protein (YFP), Venus and FlAsH and the derivatives thereof.

8. A host cell, containing the vector of any one of claims 4 to 7.

9. The host cell of claim 8, which is a mammalian cell.

10. An IP₃ indicator for detecting IP₃ in a sample, containing the protein of claim 1.

11. The IP₃ indicator of claim 10, wherein the protein of claim 1 is fused to a fluorescent protein or a photoprotein.

12. The IP₃ indicator of claim 11, which is used for detecting IP₃ by the FRET method using a combination of the protein of claim 1 labeled with one fluorescent molecule of a combination of two fluorescent molecules applicable to the FRET method, and a protein containing at least the IP₃-binding domain of the IP₃ receptor labeled with the other fluorescent molecule of the above combination.

13. The IP₃ indicator of claim 12, wherein the combination of two fluorescent molecules is a combination of Venus and CFP, a combination of YFP and CFP, or a combination of FlAsH and CFP.

14. An antibody or a functional fragment thereof, specifically recognizing the protein of claim 1.

15. The antibody or the functional fragment thereof of claim 14, which is a rabbit polyclonal antibody or a fragment thereof.

16. A method for measuring IP₃ concentration in a sample, comprising:
(i) preparing a protein complex comprising an IRBIT or a protein that contains the IP₃ receptor-binding domain of the IRBIT and a protein that contains at least the IP₃ binding domain of the IP₃ receptor,
(ii) allowing the protein complex to contact with the sample, and
(iii) quantifying a free IRBIT or a protein containing the IP₃ receptor-binding domain of the IRBIT and/or the complex.

17. The method of claim 16, wherein the quantification in (iii) above is performed utilizing an immunochemical reaction system using an anti-IRBIT antibody and/or an anti-IP₃ receptor antibody.

18. The method of claim 16 or 17, wherein the quantification in (iii) above is performed by previously labeling an IRBIT or a protein containing the IP₃ receptor-binding domain of the IRBIT or a protein containing at least the IP₃ binding domain of the IP₃ receptor, and then measuring signals originating from the labeled protein.

19. The method of claim 18, wherein the label is a fluorescent label.

20. The method of claim 16, wherein the quantification of (iii) above is performed by the FRET method.

21. The method of claim 20, which uses an IRBIT or a protein containing the IP₃ receptor-binding domain of the IRBIT labeled with YFP or Venus, and a protein containing at least the IP₃-binding domain of the IP₃ receptor labeled with CFP.

22. The method of claim 20, which uses an IRBIT or a protein containing the IP₃ receptor-binding domain of the IRBIT labeled with CFP, and a protein containing at least the IP₃-binding domain of the IP₃ receptor labeled with Venus or YFP.

23. A method for quantifying the intracellular IP₃ concentration of living cells, comprising:
(i) introducing into a cell both of a gene encoding an IRBIT or a protein containing the IP₃ receptor-binding domain of the IRBIT, labeled with one fluorescent molecule of a combination of two fluorescent molecules applicable to the FRET method, and a gene encoding a protein containing at least the IP₃ binding domain of the IP₃ receptor labeled with the other fluorescent molecule of the above combination, such that the genes can be expressed, and
(ii) irradiating the cells with the excitation wavelength of the fluorescent molecule on the lower wavelength side of the above two fluorescent molecules, and measuring under a fluorescence microscope the fluorescence wavelength of the molecule and the fluorescence wavelength of the other fluorescent molecule on the longer wavelength side.

24. The method of claim 23, wherein the combination comprising two fluorescent molecules applicable to the FRET method is a combination of Venus and CFP, a combination of YFP and CFP, or a combination of FlAsH and CFP.

25. A protein complex for quantifying IP₃ concentration, comprising an IRBIT or a protein containing the IP₃ receptor-binding domain of the IRBIT and a protein containing the IP₃ receptor or at least the IP₃ binding domain thereof.

26. A vector for quantifying intracellular IP₃ concentration of living cells, containing a gene encoding an IRBIT or a protein containing the IP₃ receptor-binding domain of the IRBIT, labeled with one fluorescent molecule of a combination of two fluorescent molecules applicable to the FRET method, and a gene encoding a protein containing at least the IP₃ binding domain of the IP₃ receptor labeled with the other fluorescent molecule of the above combination such that these genes can be expressed.

27. The vector of claim 26, wherein the combination comprising two fluorescent molecules is a combination of Venus and CFP, a combination of YFP and CFP, or a combination of FlAsH and CFP.
